# FASCICULE DE BREVET EUROPEEN

(11) **EP 3 094 717 B1**
(45) Date de publication et mention de la délivrance du brevet: **16.01.2019**
(21) Numéro de dépôt: 15707418.8
(22) Date de dépôt: 16.01.2015
(51) Int. Cl.: C12N 1/16, C12N 9/88, C12N 9/12, C12P 7/06, C12N 15/81

(54) **LEVURES MODIFIÉES POUR UTILISER LE DIOXYDE DE CARBONE**
MODIFIZIERTE HEFEN ZUR VERWENDUNG KOHLENDIOXID
YEASTS MODIFIED TO USE CARBON DIOXIDE

(30) Priorité: 16.01.2014 FR 1450349
(43) Date de publication de la demande: 23.11.2016
(73) Titulaire: Institut National De La Recherche Agronomique, 75007 Paris (FR); Institut National des Sciences Appliquées de Toulouse, 31077 Toulouse Cedex 4 (FR); Centre National de la Recherche Scientifique, 75016 Paris (FR)
(72) Inventeur: POMPON, Denis, F-31320 Pechabou (FR); PÂQUES, Frédéric, F-63000 Clermont-Ferrand (FR); LESAGE, Julie, F-31450 Belberaud (FR); GUILLOUET, Stèphane, F-31290 Vallegue (FR); BONNOT, Florence, F-81710 Saix (FR); MARC, Jillian, F-31200 Toulouse (FR); GORRET, Nathalie, F-31290 Vallegue (FR); BIDEAUX, Carine, F-31810 Le Vernet (FR); BOUTONNET, Christel, F-31860 Pins Justaret (FR)
(74) Mandataire: Be IP
(86) Numéro de dépôt international: PCT/IB2015/050346
(87) Numéro de publication internationale: WO 2015/107496

(56) Documents cités:
- WO-A1-2008/028019
- WO-A1-2009/036095
- WO-A1-2013/066848
- WO-A2-2008/135206
- CHARI A ET AL: "Cellular strategies for the assembly of molecular machines", TRENDS IN BIOCHEMICAL SCIENCES, ELSEVIER, HAYWARDS, GB, vol. 35, no. 12, décembre 2010 (2010-12), pages 676-683, XP027510134, ISSN: 0968-0004, DOI: 10.1016/J.TIBS.2010.07.006 [extrait le 2010-08-19]
- ANDREAS BRACHER ET AL: "Crystal structure of a chaperone-bound assembly intermediate of form I Rubisco", NATURE STRUCTURAL & MOLECULAR BIOLOGY, vol. 18, no. 8, 17 juillet 2011 (2011-07-17), pages 875-880, XP055139803, ISSN: 1545-9993, DOI: 10.1038/nsmb.2090
- CUIMIN LIU ET AL: "Coupled chaperone action in folding and assembly of hexadecameric Rubisco", NATURE, vol. 463, no. 7278, 14 janvier 2010 (2010-01-14), pages 197-202, XP055139806, ISSN: 0028-0836, DOI: 10.1038/nature08651 cité dans la demande
- VÍCTOR GUADALUPE-MEDINA ET AL: "Carbon dioxide fixation by Calvin-Cycle enzymes improves ethanol yield in yeast", BIOTECHNOLOGY FOR BIOFUELS, BIOMED CENTRAL LTD, GB, vol. 6, no. 1, 29 août 2013 (2013-08-29), page 125, XP021161929, ISSN: 1754-6834, DOI: 10.1186/1754-6834-6-125 cité dans la demande
- Hung-Chun Chang: "Mechanisms of De Novo Multi-domain Protein Folding in Bacteria and Eukaryotes", , 2007, XP055187947, Extrait de l'Internet: URL:http://edoc.ub.uni-muenchen.de/6520/1/ Chang_Hung-Chun.pdf [extrait le 2015-05-07]
- PIERRE GOLOUBINOFF ET AL: "GroE heat-shock proteins promote assembly of foreign prokaryotic ribulose bisphosphate carboxylase oligomers in Escherichia coli", NATURE, vol. 337, no. 6202, 5 janvier 1989 (1989-01-05), pages 44-47, XP055187918, ISSN: 0028-0836, DOI: 10.1038/337044a0
- TABITA F R ET AL: "Function, structure, and evolution of the RubisCO-like proteins and their RubisCO homologs", MICROBIOLOGY AND MOLECULAR BIOLOGY REVIEWS, AMERICAN SOCIETY FOR MICROBIOLOGY, US, vol. 71, no. 4, 1 December 2007 (2007-12-01), pages 576-599, XP002701238, ISSN: 1092-2172, DOI: 10.1128/MMBR.00015-07

### SEQUENCE LISTING

<110> INSTITUT NATIONAL DE LA RECHERCHE AGRONOMIQUE INSTITUT NATIONAL DES SCIENCES APPLIQUEES DE TOULOUSE CENTRE NATIONAL DE LA RECHERCHE SCIENTIFIQUE
<120> LEVURES MODIFIÉES POUR UTILISER LE DIOXYDE DE CARBONE
<130> VMA-sf-539-169PCT
<150> FR 14 50349
   <151> 2014-01-16
<160> 11
<170> PatentIn version 3.5
<210> 1
   <211> 1537
   <212> DNA
   <213> Synechococcus elongatus
<220>
   <221> misc_feature
   <222> (1) .. (64)
   <223> bras de recombinaison
<220>
   <221> misc_feature
   <222> (65) .. (1483)
   <223> ORF pour RbcL
<220>
   <221> misc_feature
   <222> (1485) .. (1537)
   <223> bras de recombinaison
<400> 1
   actagtttac ttcttgctca ttagaaagaa agcatagcaa tctaatctaa gttttaatta      60
   caaaatgcct aagactcaat cggctgccgg ttacaaggca ggtgtaaaag attacaaact     120
   aacttattat actccagatt atacacccaa agacactgac ttactagccg cctttcgctt     180
   ttcgccccag ccaggtgttc cagctgatga agctggtgct gctatcgctg ctgaatcttc     240
   tactggtact tggactactg tttggactga tttattaact gatatggaca gatacaaagg     300
   caaatgttac catattgaac cggttcaagg tgaggaaaat tcttattttg cttttatcgc     360
   ataccctcta gatctttttg aagagggttc tgttactaat atcttaactt ctatcgtcgg     420
   taatgtcttt ggctttaagg ccattcgtag cctacgtctt gaagacatca ggtttccagt     480
   tgctttagtt aaaacttttc aaggtccacc acatggtatc caagtagaac gggatctttt     540
   aaataaatat ggcagaccga tgctcgggtg cacgattaag ccgaagctcg ggctttctgc     600
   taaaaattat ggtagagctg tttatgaatg tttacgtggt ggtttagatt ttactaaaga     660
   tgatgaaaat atcaattctc aaccgttcca gcgttggcgg gaccgattcc tctttgtggc     720
   cgacgcgatc cataaatctc aagctgaaac tggtgaaatc aaaggtcatt atttaaatgt      780
   aacggcgcct acatgtgaag aaatgatgaa gcgagcagaa tttgctaagg aactaggtat      840
   gcctatcatc atgcatgatt ttttaactgc tggttttact gctaatacta ctttagctaa      900
   atggtgccgg gacaatggag tcctattaca tatccatcgt gccatgcacg cggtcattga      960
   tcgtcaaagg aatcatggta tccattttag agttttagct aaatgtttaa gattatctgg     1020
   tggtgatcat ttacattctg gtactgtcgt gggaaagctt gagggtgaca aggcatctac     1080
   attaggtttt gttgatttaa tgagagaaga tcatatcgaa gctgatagat ctagaggtgt     1140
   tttttttact caagactggg cgtcgatgcc gggggtgctc ccagttgctt ctggtggtat     1200
   ccatgtttgg cacatgccgg cgttagttga aatctttggt gatgattctg ttttacaatt     1260
   tggtggtggt actttaggtc atccatgggg taatgcacca ggtgctactg ctaatagagt     1320
   tgctttagaa gcttgtgttc aagctagaaa tgaaggtaga gatttatata gagagggtgg     1380
   tgatatttta agggaagcag gtaaatggtc gcctgaactg gcagccgccc tcgatttatg     1440
   gaaagaaatc aaatttgaat ttgaaactat ggataaatta taagaattcg cgggggatct     1500
   cccatgtctc tactggtggt ggtgcttctt tggtacc                              1537
<210> 2
   <211> 454
   <212> DNA
   <213> Synechococcus elongatus
<220>
   <221> misc_feature
   <222> (1) .. (64)
   <223> bras de recombinaison
<220>
   <221> misc_feature
   <222> (65) .. (400)
   <223> ORF pour Rbcs
<220>
   <221> misc_feature
   <222> (401) .. (454)
   <223> bras de recombinaison
<400> 2
   actagtttac ttcttgctca ttagaaagaa agcatagcaa tctaatctaa gttttaatta        60
   caaaatgtca atgaagacgc tccctaaaga aaggagattt gaaacgtttt catatctgcc       120
   ccctctctct gatagacaaa tcgctgctca aatcgaatat atgatcgaac aaggttttca       180
   tccattaatc gaatttaatg aacattctaa tccagaagaa ttttattgga ctatgtggaa       240
   gctccctctt tttgattgta aatctcctca acaggtttta gatgaagtga gagagtgtag       300
   atctgaatat ggtgattgtt atatcagagt tgctggtttt gataatatca aacaatgtca       360
   aactgtttct tttatcgttc atagacctgg aagatactaa gaattcgcgg gggatctccc       420
   atgtctctac tggtggtggt gcttctttgg tacc                                   454
<210> 3
   <211> 604
   <212> DNA
   <213> Synechococcus elongatus
<220>
   <221> misc_feature
   <222> (1) .. (64)
   <223> bras de recombinaison
<220>
   <221> misc_feature
   <222> (65) .. (550)
   <223> ORF pour RbcX
<220>
   <221> misc_feature
   <222> (551) .. (604)
   <223> bras de recombinaison
<400> 3
   actagtttac ttcttgctca ttagaaagaa agcatagcaa tctaatctaa gttttaatta       60
   caaaatgcaa tttatgggta ctgcttctag gatggcgtcg acgcaacggg ccaagcctat      120
   ggagatgccg aggattagcc gtgatactgc tagaatgtta gttaattatt taacttatca      180
   agctgtttgt gttatcagag atcaattagc tgaaactaat ccagctggtg cctatagatt      240
   acaagttttt tctgctgaat tttcttttca agatggtgaa gcttatttag ctgctttatt      300
   aaatcatgat agagaattag gactaagggt gatgacggta agggaacatt tagctgaaca      360
   tattctagat tatcttccag aaatgacgat tgcccaaatt caagaggcca acattaacca      420
   tagaagagca cttttagaaa ggttaacagg ccttggggct gagccatctt taccggaaac      480
   ggaggtctca gacagaccct cagattctgc tactccagat gatgcttcta atgcttctca      540
   tgctgattaa gaattcgcgg gggatctccc atgtctctac tggtggtggt gcttctttgg      600
   tacc                                                                   604
<210> 4
   <211> 1120
   <212> DNA
   <213> Synechococcus elongatus
<220>
   <221> misc_feature
   <222> (1) .. (64)
   <223> bras de recombinaison
<220>
   <221> misc_feature
   <222> (65) .. (1066)
   <223> ORF pour Prk
<220>
   <221> misc_feature
   <222> (1067) .. (1120)
   <223> bras de recombinaison
<400> 4
   actagtttac ttcttgctca ttagaaagaa agcatagcaa tctaatctaa gttttaatta 60
   caaaatgtct aaaccagata gagttgtttt aatcggtgtt gctggtgatt ctggttgtgg 120
   taaatctaca tttcttaaca ggttagctga tttatttggt actgaattaa tgactgttat 180
   ttgtttagat gattatcatt cgttagatcg taaaggcaga aaggaagcgg gtgtaactgc 240
   tttagatcct agagctaata attttgattt aatgtatgaa caagttaaag ctttaaaaaa 300
   tggtgaaact atcatgaaac caatctataa tcatgaaact ggtttaatcg atccacctga 360
   aaagatcgag ccaaacagaa ttattgtaat tgaagggtta cacccacttt atgacgaacg 420
   agttcgcgaa cttttagatt tttctgttta tttagatatc gatgatgaag ttaaaatcgc 480
   ttggaaaatc caaagagata tggccgaaag aggtcattct tatgaagatg ttttagcctc 540
   aattgaggct agaaggccag attttaaagc atatattgaa ccgcaacggg gacacgctga 600
   tatcgttatt cgtgtaatgc ccactcaact tatcccgaat gacactgaga ggaaagtcct 660
   aagggtacaa ttaatccaga gagaaggaag ggatggattt gaaccagctt atttatttga 720
   tgaaggttct acaattcaat ggacgccttg tggcagaaag ttaacatgta gctatcctgg 780
   cattcgctta gcttatggtc cagatactta ttatggtcat gaagtttctg tccttgaagt 840
   ggatggacaa tttgaaaatt tagaagaaat gatttacgtt gaaggtcatt tatctaaaac 900
   tgatactcaa tattatggtg aattaactca tctactttta caacacaaag attatccagg 960
   ttctaataat ggtactggtt tattccaagt gctaacgggt ctcaagatgc gggccgccta 1020
   tgaaaggtta acttctcaag ctgctccagt tgctgcttct gtttaagaat tcgcggggga 1080
   tctcccatgt ctctactggt ggtggtgctt ctttggtacc 1120
<210> 5
   <211> 939
   <212> DNA
   <213> Rhodobacter sphaeroides
<400> 5
   cacacactaa attaccggat caattcgggg gatccatgtc taaaaaacat ccaatcatca 60
   gtgtaacggg cagttcgggg gcgggaactt caactgtcaa acatactttt gatcaaattt 120
   ttcgtcgtga aggcgtcaag gcagtttcta tcgaaggtga tgcttttcat agatttaata 180
   gagctgatat gaaagcagag ctcgatcggc gatacgcggc aggtgatgct actttttctc 240
   atttttctta tgaagctaat gaactgaaag agctggagag agtgtttcga gaatacgggg 300
   aaacggggca gggtcggaca cgtacttatg ttcatgatga tgctgaagcg gcgcggactg      360
   gagtcgcgcc aggtaatttt actgattgga gagattttga ttctgattct catttattat      420
   tttatgaagg tttacatgga gctgtagtta actctgaggt taacatcgcg ggtctggctg      480
   atttaaaaat cggtgttgtt ccagttatca atttagaatg gattcaaaaa atccatagag      540
   atagagcgac taggggttat actactgaag cagtcacgga tgtcattcta cgtagaatgc      600
   atgcttatgt tcattgtatc gttccacaat tttctcaaac tgatatcaat tttcaacggg      660
   tgccggtggt ggatacctct aatcctttta ttgctagatg gattcctact gctgatgagt      720
   cagtggtagt aattcgtttt cggaatcctc gtggaattga ctttccttat cttacatcta      780
   tgattcatgg tagttggatg tcgagagcca atagtattgt cgtccctgga aataagctgg      840
   acctggccat gcagctcatt ctcacgccgc tgattgacag gctggttcga gaatcaaaag      900
   ttgcttaact gcaggagggc cgcatcatgt aattagtta                             939
<210> 6
   <211> 942
   <212> DNA
   <213> Rhodopseudomonas palustris
<400> 6
   cacacactaa attaccggat caattcgggg gatccatgtc tagaaaacat ccaatcatct       60
   ctatcactgg ttcttctggt gctggtacta cttctgttaa aaaaactttt gaacaaatct      120
   ttagaagaga aaatgttaat gctgctttta tcgaaggtga tgcttttcat cgatataata      180
   gagttgatat gagaaataaa atggctgaag aagctgaaag aggtaataga catttttctc       240
   atttttctcc agaaactaat ttatttgaag agttagaaca gacttttaga tcttatgctg      300
   aaactggtat cggtagaact agacattatg ttcatgatga tgaagaagct gcattacatg      360
   gagtcccgcc aggtaatttt actcaatggc aggacctccc tgccaatagt gatttattat      420
   tttatgaagg tttacatggt gctgttatca ctgaaaaagt taatgttgct caacatgctg      480
   atttaaaaat cggtgttgtt cctgttatca atttagaatg gattcaaaaa ttacatcgcg      540
   accgagcggc cagggggtac tcgactgagg ctgtcactga tacaatcctt agaagaatgc       600
   cagattatgt tcattatatc gttccacaat ttgctgaaac tgatatcaat tttcaaagag      660
   ttccaactgt tgatacttct aatccattta tcgctagatg gattccaact gctgatgaat      720
   ctatggttgt tatcagattt aaaaatccaa gaggaattga ctttgcgtac ctcttatcta      780
   tgatccaagg ttcttttatg tctagagcta attctatcgt tatccatggt gctaaattag      840
   atctggcgat gcagcttatt cttactccat taatcatgca gcttatcgat agaaaaagat      900
   ctatgaaata actgcaggag ggccgcatca tgtaattagt ta                         942
<210> 7
   <211> 1275
   <212> DNA
   <213> Euglena gracilis
<400> 7
   cacacactaa attaccggat caattcgggg gatccatggc tgtttgtact gtttatacta      60
   tccctacaac aactcatctt ggttcatctt ttaatcagaa taataaacag gtcttcttta     120
   attataaaag atcatcatca tcaaataaca ccctgttcac aactaggcca tcttatgtta     180
   tcacttgttc tcaacaacaa actatcgtta tcggtttagc tgctgattct ggttgtggta     240
   aatctacttt tatgagaaga ttaacttctg tttttggtgg tgctgctgaa ccaccaaaag     300
   gaggcaaccc tgatagcaat acgttaatct ctgatactac tactgttatt tgtctagatg     360
   acttccacag tttagataga aatggtagaa aagttgaaaa agttactgct ttagatccca     420
   aggcgaacga ctttgactta atgtatgaac aagttaaagc tttaaaagaa ggtaaagctg     480
   ttgataaacc aatttacaac cacgtgtcgg gcctgctcga ccctccggaa ctcattcagc     540
   cacccaaaat tctggtcatt gagggattac atcctatgta cgatgctaga gttagagagc     600
   tactcgactt ctctatttac ctcgacatct caaatgaagt taaatttgct tggaaaatcc     660
   aaagagatat gaaagaaaga ggtcattctt tagaatctat caaagcttct atcgaatcta     720
   gaaagcctga tttcgatgcg tacatcgatc cacaaaaaca acatgctgat gttgttattg     780
   aagtgctccc taccgaactc attccggatg atgatgaagg taaagtttta agagttagaa     840
   tgatccaaaa agaaggtgtt aaatttttca acccggtgta cctttttgat gaaggttcta    900
   ctatctcttg gattccatgt ggtagaaaat taacttgttc ttatccaggt atcaagtttt     960
   cgtacgggcc agacacattt tacggtaatg aagttactgt tgttgaaatg gatggtatgt    1020
   tcgatcgttt agatgagtta atctacgttg agtcacactt atcgaatctg tcaactaaat    1080
   tttatggtga agttactcaa caaatgttaa agcatcagaa ttttcctggc agtaataatg    1140
   gaactggttt ttttcaaact atcatcggtc ttaaaattag ggatttattt gaacaattag    1200
   ttgcttctag atctactgct actgctactg ctgctaaagc ttaactgcag gagggccgca    1260
   tcatgtaatt agtta                                                     1275
<210> 8
   <211> 1434
   <212> DNA
   <213> Spinacia oleracea
<400> 8
   cacacactaa attaccggat caattcgggg gatccatgag atatgatcaa ctcattcatg      60
   agggtccgag acagtcatcg ccttcgcttc tgcaactcgc ttctgtttct gctactgtct     120
   tttgtgctgt ttttggtatc ggttatactt tttctggtgg tttagttgaa aattatgcaa     180
   ctactcgtcc tgtgcagacg caacccgctg cgttaatcct tccaaaagct gtcaggtatg     240
   caggagtatc acaggggccg caggtagagt cacgggaagc cagaacagct ttacatgctg     300
   ctgctactgg tactgttaat agagactcaa cgctacaaag gccaaaagtt gatccaaaaa     360
   aaactgtttt aatcggggtc gcagctgatt ctggttgtgg taaatctact tttatgagaa     420
   gactaactgg tatttttgga ggtggaaaac ccacacctct tggaggtggg tttggtactg     480
   gtggttggga aactaatact ttagtttctg ataaaactac tgttatgtgt ttagatgatt     540
   atcatttaaa tgatagagct ggtcgtaaag taacgggtct gactgccctt gatcaaagag     600
   aaaataattt tgatttaatg tttgaacaaa tgtcttcttt aaaaagagga gaaactatcg     660
   ctaaaccaat ctataatcat gttaatggta ctttagatac tccagaagag atcgctccag     720
   cttctatcat gatcattgaa ggccttcacc ccctgctaga tgaccgtgtg gctggtttat     780
   tagatttttc tatctattta gatatctctg atagagttaa atttgcttgg aaaatccaaa     840
   gagatatggc ggagaggggt tgggctttag aagatatcaa aaaagatatc gaaaaaagaa     900
   aaccagattt tgataaatat gttgctccac aaagggctaa agctgatatg gtaattgagg     960
   tgctccctag taggcttgca cccccaaaag atgaaacggc gcccctcgaa tacctccgtg    1020
   tgcgattaat ccaaaaaact actactaaac attttgatcc agtttattta atcgaaaaag    1080
   gttcttctgt tacttggaaa ccatgtggtg acaacctgca atgcgagtac ccaggattac    1140
   agttagctta ttatactgaa gaatatatgg ggcatccggc cgaagtgctc gaaatggacg    1200
   gtgttatcca taatttaaaa gaaggtttat atgttgaaaa atttttacat aatactggtg    1260
   ctaaagaatt tggagaactc acgcaggaac tccttaaagg tcagaattct ccaggtggtg    1320
   ataatggtac tggttttatg caaactttag ctgctttaaa aatcagggaa atttacgaaa    1380
   gggctactgg tgaaaaagct taactgcagg agggccgcat catgtaatta gtta          1434
<210> 9
   <211> 1768
   <212> DNA
   <213> Synechococcus elongatus
<220>
   <221> misc_feature
   <222> (1) .. (50)
   <223> bras de recombinaison
<220>
   <221> misc_feature
   <222> (51) .. (1718)
   <223> ORF
<220>
   <221> misc_feature
   <222> (51) .. (1718)
   <223> ORF de GroEL1
<220>
   <221> misc_feature
   <222> (1719) .. (1768)
   <223> bras de recombinaison
<400> 9
   tttaaaacac caagaactta gtttcgaata aacacacata aacaaacaaa atggctaaat     60
   taatcttatt tcatgaagat tcaagacaag cattagaaag gggtgttaat gctttagcta    120
   atgctgttaa agttacttta ggtccaagag gtagaaatgt tttattagaa aaaaaatttg    180
   gtgctccaga aatcatcaat gatggtgttt ctatcgctaa agaaatcgaa ttagaagatc    240
   cacatgaaaa tgcaggtgca agactagttc aagaagttgc tgctaaaact aaagaaatcg    300
   ctggtgatgg tactactact gctactgttt tagctcaagc tatcgttaga gaaggtttaa    360
   ctaatgttgc tgctggtgct aatccaatcg ttttaagaag aggtatcgaa aaagctgttg    420
   ctactttagt tgaagctatc gctgctaaag ctcaaccagt tgctgatgaa gctgctatca    480
   gatctatcgc tgctgtttct gctggtaatg atgatgaagt tggtcaaatg atcgctgatg    540
   ctgttgctaa agttactaaa gatggtgtta tcacagttga agaatctaaa tctttagcta    600
   ctgaattaga agtcgttgaa ggtatgcaat ttgatagagg ttatttatct ccatattttg    660
   ttactgatca agatagacaa gtagttgaat atgataatcc attaatctta ttaactgata    720
   aaaaaatcgc ttctatccaa gatttagttc cagttttaga agatgttgct agagctggta    780
   gaccattatt aatcatcgct gaagatatcg aaggtgaagc tttagctact ttagttgtta    840
   ataaagctag aggtgtttta aatactgttg ctgttaaagc tccagctttt ggtgatagaa    900
   gaaaagctat cttacaagat atcgctgttt taactggtgg tcaagttatc tctgaagaag    960
   ttggtttatc tttagctgat gctaattctt ctgttttagg taaagctcaa aaaatcacta   1020
   tctctaaaga tactactatc atcgttgctg gtgatgaaaa taaagctgat gttgctgcta   1080
   gaatcgctca aatcagaaga tctttagaag aaactgattc tgattatgat agagaaaaat   1140
   tacaagaaag aatcgctaaa ttagctggtg gtgttgctgt tatcaaagtt ggtgctccaa   1200
   ctgaaactga attaaaaaat agaaaattaa gaatcgaaga tgctttaaat gctactagag   1260
   ctgctatcga agaaggagtt gttccaggtg gtggtactac tttattacat ttagcttctg   1320
   ctttaacttc tttacaagct tctttaactg ttgctgatga aaaattaggt gttgaaatcg   1380
   ttgctagagc tttagaagct ccattaagac aaattgctga taatgctggt gcagaaggtt   1440
   ctgttgttgt cgaaaaatta agagataaag attttaattt tggttataat gctttaactg   1500
   gtcaatatga agatttagtt gcttctggta tcttagatcc agctaaagtt gttagatctg   1560
   ctttacaaga tgctgcttct gttgcttctt taatcttaac tactgaagtt ttagttgttg   1620
   atcaacctga accagaacca gctatgcctg ctggtggtga tatgggtggt atgggtggta   1680
   tgggtatgcc tggtatgggt ggtatgggta tgatgtaaga attcacttct aaataagcga   1740
   atttcttatg atttatgatt tttattat                                      1768
<210> 10
   <211> 1735
   <212> DNA
   <213> Synechococcus elongatus
<220>
   <221> misc_feature
   <222> (1) .. (50)
   <223> bras de recombinaison
<220>
   <221> misc_feature
   <222> (51) .. (1685)
   <223> ORF de GroEL2
<220>
   <221> misc_feature
   <222> (1686) .. (1735)
   <223> bras de recombinaison
<400> 10
   tttaaaacac caagaactta gtttcgaata aacacacata aacaaacaaa atggctaaaa     60
   gaatcatcta taatgaaaat gctagaagag ctttagaaaa aggtatcgat atcttagctg    120
   aagctgttgc tgttacttta ggtccaaaag gtagaaatgt cgtcttagaa aagaaatttg    180
   gtgcaccaca aattatcaat gatggtgtta ctatcgctaa agaaatcgaa ttagaagatc    240
   atatcgaaaa tactggtgtt gctttaatca gacaagcagc ttcaaaaaca aatgatgctg    300
   ctggtgatgg tactactact gctactgttt tagctcatgc tgttgtcaaa gaaggtttaa    360
   gaaatgttgc tgctggtgct aatgctatct tattaaaaag aggtatcgat aaagctacaa    420
   attttcttgt cgaacaaatt aaatcacatg ctcgtccagt cgaagattct aaatctatcg    480
   cacaagttgg tgcaatctct gctggtaatg attttgaagt tggtcaaatg atcgctgatg    540
   ctatggataa agttggtaaa gaaggtgtta tctctttaga agaaggtaaa tctatgacta    600
   ctgaattaga agttactgaa ggtatgcgtt ttgataaagg ttatatctct ccatattttg    660
   ctactgatac tgaaagaatg gaagccgtct ttgatgaacc atttatctta atcactgata    720
   aaaaaatcgg attagttcaa gatcttgtcc cagttttaga acaagttgct agagctggta    780
   gaccattagt tattatcgca gaagatatcg aaaaagaagc tttagctact ttagttgtta    840
   atagattaag aggtgtctta aatgttgcag ctgtcaaagc tccaggtttt ggtgatagaa    900
   gaaaagctat gttagaagat atcgctgttc ttacaggtgg tcaacttatc acagaagatg    960
   ctggtttaaa attagatact actaaattag atcaattagg taaagctaga agaatcacta   1020
   tcactaaaga taatactact atcgttgctg aaggtaatga agctgctgtt aaagctagag   1080
   tcgatcaaat tagaaggcaa attgaagaaa cagaaagctc ttatgataaa gaaaagttac 1140
   aagaaagatt agctaaatta tctggtggtg tcgcagttgt caaagttggt gctgctactg 1200
   aaactgaaat gaaagataga aaattaagat tagaagatgc tatcaatgct actaaagctg 1260
   ctgttgaaga aggtatcgtt ccaggtggtg gtactacttt agctcattta gctccacaat 1320
   tagaagaatg ggcaactgct aatttatctg gtgaagaatt aactggtgct caaatcgttg 1380
   ctagagcttt aactgctcca ttaaaaagaa tcgctgaaaa tgctggttta aatggtgctg 1440
   ttatctctga aagagtcaaa gaattaccat ttgatgaagg ttatgatgca tcaaataatc 1500
   aatttgttaa tatgtttact gctggtattg ttgatccagc taaagttaca agatcagctt 1560
   tacaaaatgc tgcttctatc gctgctatgg ttttaactac tgaatgtatc gttgttgata 1620
   aaccagaacc aaaagaaaaa gctccagctg gtgctggtgg tggtatgggt gattttgatt 1680
   attaagaatt cacttctaaa taagcgaatt tcttatgatt tatgattttt attat 1735
<210> 11
   <211> 412
   <212> DNA
   <213> Synechococcus elongatus
<220>
   <221> misc_feature
   <222> (1) .. (50)
   <223> bras de recombinaison
<220>
   <221> misc_feature
   <222> (51) .. (362)
   <223> ORF de GroES
<220>
   <221> misc_feature
   <222> (363)..(412)
   <223> bras de recombinaison
<400> 11
   attcctctag tcttgcaaaa tcgatttaga atcaagatac cagcctaaaa atggctgccg 60
   tctcattatc tgtttctact gttactccat taggtgatag agtttttgtt aaagttgctg 120
   aagctgaaga aaaaactgct ggtggtatca tcttaccaga taatgctaaa gaaaaaccac 180
   aagtcggtga aattgtcgct gttggtccag gtaaaagaaa tgatgatggt tcaagacaag 240
   ctccagaagt taaaatcggt gataaagttt tatattctaa atatgctggt actgatatta 300
   aattaggtaa tgatgattat gttcttttat ctgaaaaaga tatcttagct gttgtcgctt 360
   aagaattcat catgtaatta gttatgtcac gcttacattc acgccctccc cc 412

## Description

L'invention est relative à la création de souches de levure modifiées dans le but de leur permettre d'utiliser le dioxyde de carbone comme source de carbone.

L'activité humaine et industrielle génère des quantités croissantes de dioxyde de carbone qui est un des facteurs principaux de l'effet de serre, responsable d'un réchauffement global de la planète susceptible d'induire des changements climatiques majeurs.

La capture du dioxyde de carbone et sa transformation en composés organiques sont naturellement effectuées par certains organismes, notamment dans le cadre de la photosynthèse.

La photosynthèse comprend deux phases : pendant la première phase interviennent des réactions photochimiques à l'issue desquelles l'énergie lumineuse est convertie en énergie chimique sous forme d'ATP et de NADPH/NADH ; au cours de la seconde phase, dénommée cycle de Calvin, cette énergie chimique est utilisée pour incorporer le carbone provenant du dioxyde de carbone dans des molécules organiques.

L'enzyme clé du cycle de Calvin est le complexe RuBisCO (Ribulose 1,5 bisphosphate carboxylase/oxygénase) qui convertit le ribulose 1,5 diphosphate en 2 molécules de 3-phosphoglycérate par capture d'une molécule de dioxyde de carbone.

Il existe plusieurs formes de RuBisCO (TABITA et al., J Exp Bot, 59, 1515 - 24, 2008), dont les plus représentées sont la forme I et la forme II. La forme I est constituée de deux types de sous-unités : des grandes sous-unités (RbcL) et des petites sous-unités (RbcS). Le complexe enzymatique fonctionnel est un hexadécamère constitué de huit sous-unités L et huit sous-unités S. L'assemblage correct de ces sous-unités nécessite en outre l'intervention d'au moins une chaperonne spécifique : RbcX (LIU et al., Nature, 463, 197-202, 2010). La forme II est beaucoup plus simple : il s'agit d'un dimère formé de deux sous-unités RbcL identiques.

Le ribulose 1,5 diphosphate, substrat de la RuBisCO, est formé par réaction du ribulose 5-phosphate avec l'ATP ; cette réaction est catalysée par une phosphoribulokinase (PRK). On connait 2 classes de PRKs : les enzymes de classe I qui se rencontrent chez les protéobactéries sont des octamères, tandis que celles de classe II qui se trouvent chez les cyanobactéries et chez les plantes sont des tétramères ou des dimères.

Les organismes non-photosynthétiques, tels que les levures, ne possèdent ni RuBisCO ni phosphoribulokinase, mais contiennent en revanche les autres enzymes du cycle de Calvin, car celles-ci interviennent également dans le métabolisme général des pentoses.

Il a été proposé d'introduire la RuBisCO et la PRK chez des levures afin de reconstituer un cycle de Calvin, et de leur permettre d'utiliser le dioxyde de carbone. Ainsi, GUADALUPE-MEDINA et al. (Biotechnology for Biofuels, 6, 125, 2013) rapportent que l'expression chez *Saccharomyces cerevisiae* de l'enzyme RuBisCO de forme II de *Thiobacillus denitrificans,* et de la PRK de *Spinacia oleracea* permet d'améliorer la production d'éthanol en diminuant la formation de glycérol.

Toutefois, jusqu'à présent, il n'avait pas été possible d'exprimer chez la levure une enzyme RuBisCO de forme I d'origine bactérienne. En effet, du fait de la complexité de cette forme, la reconstitution d'une enzyme fonctionnelle nécessite la coexpression dans une stoechiométrie appropriée, des sous-unités RbcL et RbcS ainsi que de la chaperonne RbcX, et l'association correcte de ces sous-unités dans le complexe enzymatique. Or, la transposition chez un organisme eucaryote de la stoechiométrie d'expression qui chez les procaryotes est assurée par l'organisation des gènes en opérons, pose des problèmes. En outre, les différences existant entre eucaryotes et procaryotes au niveau de l'environnement intracellulaire peuvent se traduire notamment par des modifications post-traductionnelles interférant avec le repliement des chaînes peptidiques constituant les sous-unités enzymatiques et/ou avec l'assemblage de ces sous-unités.

Les Inventeurs sont toutefois parvenus à exprimer chez la levure les différentes sous-unités de l'enzyme RuBisCO de forme I de *Synechococcus elongatus* et à obtenir l'assemblage de ces sous-unités pour reconstituer le complexe enzymatique, en co-exprimant ces sous-unités avec la chaperonne spécifique RbcX, et avec les chaperonnes généralistes bactériennes GroES et GroEL.

La présente invention a ainsi pour objet une cellule de levure, de préférence une cellule de *Saccharomyces cerevisiae,* transformée, selon la revendication 1.

Un caractère particulièrement original de l'invention est que les chaperonnes mentionnées aux points c), d) et e) ci-dessus appartiennent à deux organismes différents. Les chaperonnes généralistes (GroES, GroEL) proviennent de *E*. *Coli* et la chaperonne "spécifique" (RbcX) provient de *Synechococcus elongatus.* La similarité de séquence des chaperonnes (% d'acides aminés identiques dans l'alignement) est de 61 % entre GroEL1 de *S. elongatus* et GroEL de *E. coli ;* de 56 % entre GroEL2 de *S. elongatus* et GroEL de *E. coli ;* de 63 % entre GroEL1 et GroEL2 de *S. elongatus.* Un seuil de 65% d'identité distingue donc les chaperonnes généralistes de *E*. *coli* et de *S. elongatus.* Dans le présent texte, on désigne par "GroES" et "GroEL" toute protéine présentant une activité de chaperonne et ayant entre 65 et 100 % d'identité en acides aminés avec les GroES et GroEL de *E. coli* K 12, respectivement. L'activité chaperonne d'un variant des chaperonnes généralistes GroES et GroEL d'*E*. *coli* pourra être vérifiée par exemple en substituant dans les différents exemples décrits de mise en oeuvre de l'invention, la cassette d'expression codant pour GroES ou GroEL natif de *E. coli* par des variants de chaperonnes à évaluer.

La chaperonne RbcX est très éloignée de GroEL et GroES et sa séquence ne peut être alignée avec les séquences de ces deux chaperonnes. Dans le présent texte, on désigne par "RbcX" toute chaperonne de cyanobactérie présentant plus de 50% d'identité de séquence (en acides aminés) avec la chaperonne RbcX codée par la SEQ ID No : 3 et conservant l'activité de chaperonne spécifique de cette protéine (ce qui peut être vérifié dans une levure exprimant les sous unités RbcL et RbcS de la RuBisCO de *S. elongatus,* en remplaçant la cassette d'expression incluant la séquence ID N°3 de l'invention par toute autre séquence à évaluer, et en mesurant par un test *in vitro* sur des extraits cellulaires l'activité RuBisCO ainsi obtenue). De préférence, la présente invention est mise en oeuvre avec une chaperonne RbcX dont l'identité de séquence (en acides aminés) avec la chaperonne RbcX codée par la SEQ ID No : 3 est supérieure à 80%, voire supérieure à 90 %.

Selon une mise en oeuvre préférée de la présente invention, la chaperonne RbcX est une chaperonne de cyanobactérie, par exemple de *Synechococcus elongatus.*

Selon une autre mise en oeuvre préférée, au moins une des chaperonnes généralistes GroES et GroEL ne provient ni d'une cyanobactérie ni d'une autre bactérie exprimant un complexe RuBisCO.

Selon un mode de réalisation avantageux, les trois cassettes d'expression forment un bloc continu d'information génétique. Il peut également être avantageux que les cassettes d'expression des trois chaperonnes soient portées par un élément génétique épisomique unique.

L'enzyme RuBisCO de forme I d'origine bactérienne est une enzyme RuBisCO de cyanobactérie, du genre *Synechococcus* et de manière tout à fait préférée de *Synechococcus elongatus.*

Selon un mode de réalisation préféré de la présente invention, ladite cellule contient en outre une cassette d'expression f) contenant une séquence codant pour une PRK, de préférence une PRK de classe II, par exemple une PRK de *Spinnacia oleacera, Euglena gracilis,* ou *Synechococcus elongatus,* sous contrôle transcriptionnel d'un promoteur approprié.

Une large variété d'outils (promoteurs, cassettes vecteurs d'expression, méthodes de transformation) utilisables pour l'expression de gènes d'intérêt dans des cellules de levure est disponible dans l'art (pour revue cf. par exemple « Methods in Yeast Genetics » D. Amberg, D. Burke and J. Strathem, Cold Spring Harbor Laboratory Press, 2005).

Des promoteurs utilisables dans le cadre de la présente invention incluent des promoteurs constitutifs, à savoir des promoteurs qui sont actifs dans la plupart des états cellulaires et des conditions environnementales, ainsi que des promoteurs inductibles qui sont activés ou réprimés par des stimuli physiques ou chimiques exogènes, et qui induisent donc un niveau d'expression variable en fonction de la présence ou de l'absence de ces stimuli.

Pour les cassettes d'expression a) à e) on utilisera de préférence des promoteurs constitutifs, tels que par exemple TEF1, TDH3, PGI1, PGK, ADH1. De préférence, ces promoteurs seront différents d'une cassette à l'autre.

Pour la cassette d'expression f) de la PRK on utilisera de préférence un promoteur inductible. A titre d'exemple, on citera le promoteur tet O-7 dont l'expression est réprimée par la doxycycline, et donc induite par l'absence de celle-ci.

D'autres promoteurs inductibles utilisables dans le cadre de la présente invention sont notamment les promoteurs tet O-2, GAL10, GAL10-cyc1, PHO5.

Les cassettes d'expression de l'invention comprennent en outre les séquences usuelles dans ce type de construction, telles que des terminateurs transcriptionnels, et le cas échéant d'autres éléments de régulation de la transcription tels que des amplificateurs.

La stoechiométrie relative des protéines exprimées par les différentes cassettes d'expression est susceptible de jouer un rôle important dans la mise en oeuvre optimale de la présente invention. Le système de coexpression chez la levure décrit dans la partie expérimentale ci-après est particulièrement pertinent à cet égard. L'invention n'est toutefois pas limitée à l'utilisation de ce système, et elle peut être mise en oeuvre avec n'importe quelle variante d'expression des éléments mentionnés ayant des effets au moins équivalents, tels qu'ils peuvent être mesurés, par exemple, en reproduisant un des exemples décrits ci-après.

Les cassettes d'expression conformes à l'invention peuvent être insérées dans l'ADN chromosomique de la cellule-hôte, et/ou portées par un ou plusieurs réplicon(s) extra-chromosomiques.

Les souches de levure conformes à l'invention peuvent être cultivées dans les conditions usuelles de culture de souches de la même espèce. Avantageusement, ces cultures seront effectuées sous une atmosphère contenant au moins 90% de dioxyde de carbone.

La présente invention sera mieux comprise à l'aide du complément de description qui va suivre, qui se réfère à des exemples non-limitatifs décrivant l'expression d'un complexe RuBisCO et d'une PRK chez *Saccharomyces cerevisiae.*

### LEGENDE DES FIGURES

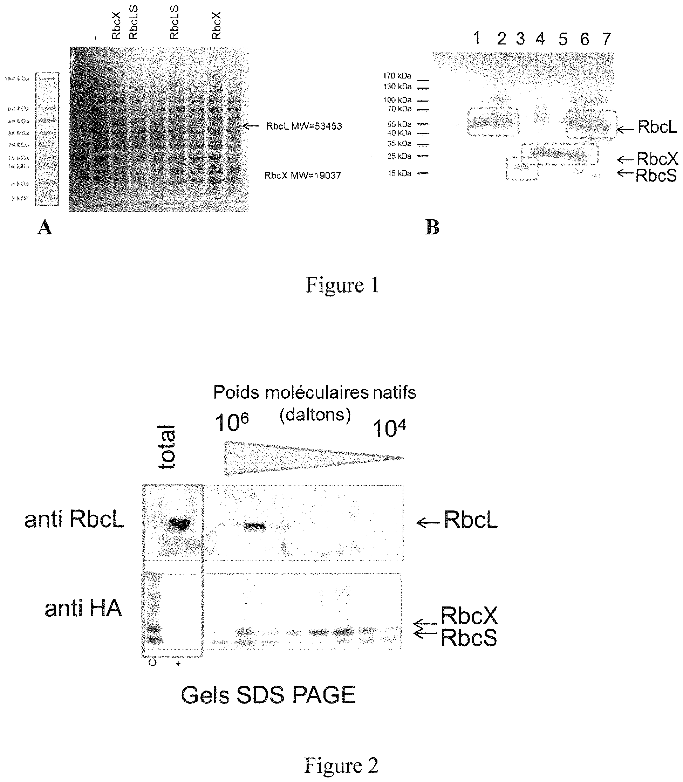

**Figure 1** : analyse de lysats totaux de souches transformées. 1A : analyse SDS-PAGE d'un lysat total des souches 11.5, 11.15, 11.7, 11.17, 11.9, 11.19, 11.5, 11.19. ; 1B : analyse par immuno-détection anti-HA d'un lysat total des souches 14.5, 14.12, 14.6, 14.7, 16.3, 16.5, 16.6.
**Figure 2** : analyse par immunodétection d'un lysat total (encadré sur la gauche de la Figure), et des fractions triées par poids moléculaire, de la souche 16.5, qui co-exprime RbcL, RbcS, et RbcX et de son contrôle, la souche 16.3 qui exprime RbcX.

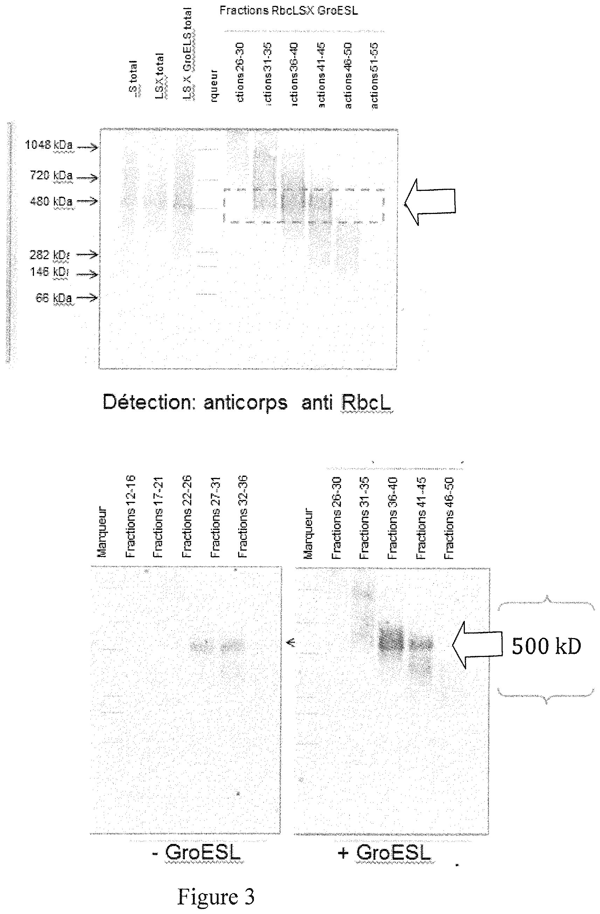

**Figure 3** : analyse sur gel non-dénaturant, suivie d'immunodétection à l'aide d'un anticorps anti-RbcL, des extraits totaux des souches 11.9, 18.3 et 22.2, et de fractions triées par poids moléculaire de la souche 22.2, qui co-exprime RbcL, RbcS, et RbcX, de *S. Elongatus* et les chaperonnes de E. *coli.* Puis, en parallèle, les fractions triées par poids moléculaire des souches 18.3 (à gauche) et 22.2 (à droite).

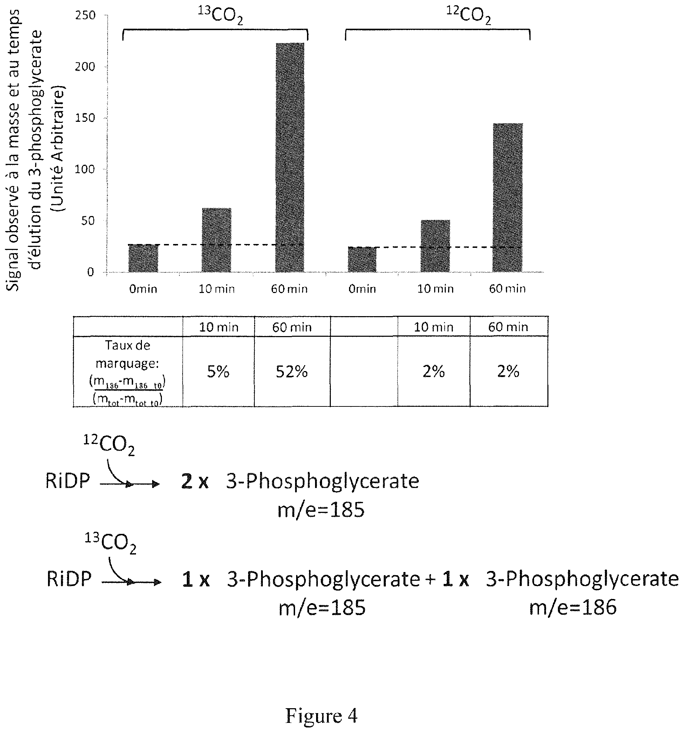

**Figure 4** : quantité de 3-phosphoglycérate détectée (m/e de 185 et 186 c'est-à-dire les ions du 3-phosphoglycérate non marqué et du 3-phosphoglycérate marqué ¹³C sur un carbone en ES⁻) obtenue à différents temps de réaction (0,10 et 60 min). A gauche sont représentés les expériences réalisées en présence de ¹³CO₂ et à droite celles réalisées en présence de ¹²CO₂.

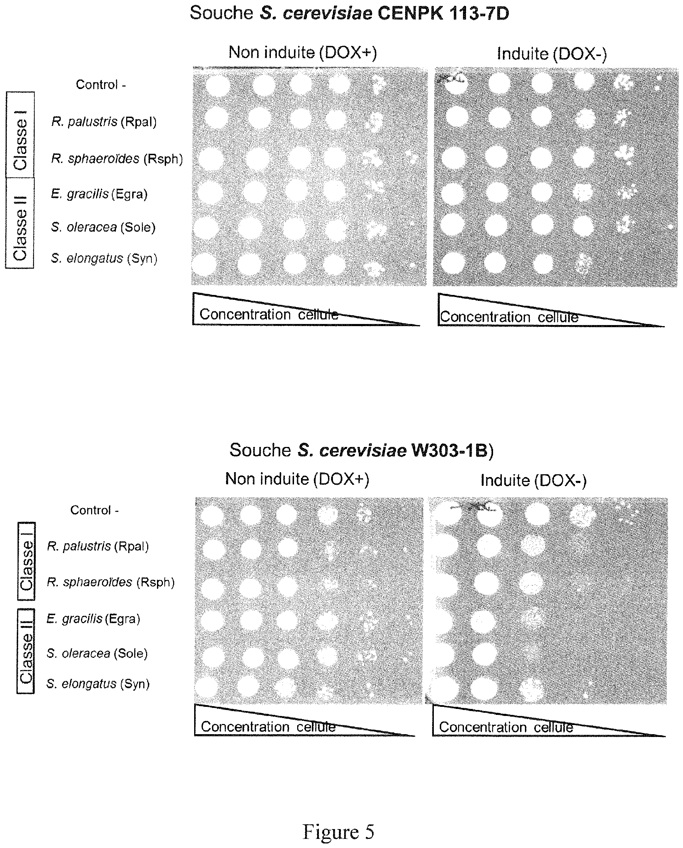

**Figure 5** : influence de l'expression des différentes PRKs sur la viabilité des cellules, en atmosphère normale. Chaque souche est cultivée en liquide sur milieu CSM sélectif avec doxycycline à 2 µg/mL. Un équivalent de 2 DO (DO à 600nm) est récupéré puis lavé 2 fois pour éliminer la doxycycline. Des dilutions au dixième sont réalisées. 10µL des dilutions sont déposées sous forme de gouttes (séries de dilutions sérielles) des suspensions de cellules, sur des boites de milieux gélosés (contenant ou non de la 2 µg/mL doxycycline) et incubées à 28°C en atmosphère normale.

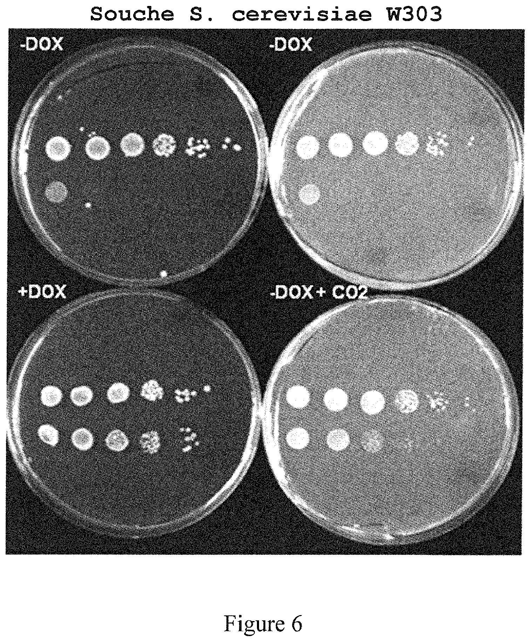

**Figure 6** : influence de l'expression des différentes PRKs sur la viabilité des cellules, en atmosphère riche en CO₂. Chaque souche est cultivée en liquide sur milieu CSM sélectif avec doxycycline à 2 µg/mL. Un équivalent de 2 DO (DO à 600nm) est récupéré puis lavé 2 fois pour éliminer la doxycycline. Des dilutions au dixième sont réalisées. 10µL des dilutions sont déposées sous forme de gouttes (séries de dilutions sérielles) des suspensions de cellules, sur des boites de milieux gélosés (contenant ou non de la 2 µg/mL doxycycline) et incubées à 28°C en sacs fermés dont l'atmosphère contient au moins 90 :10 de dioxyde de carbone/air (vol/vol).

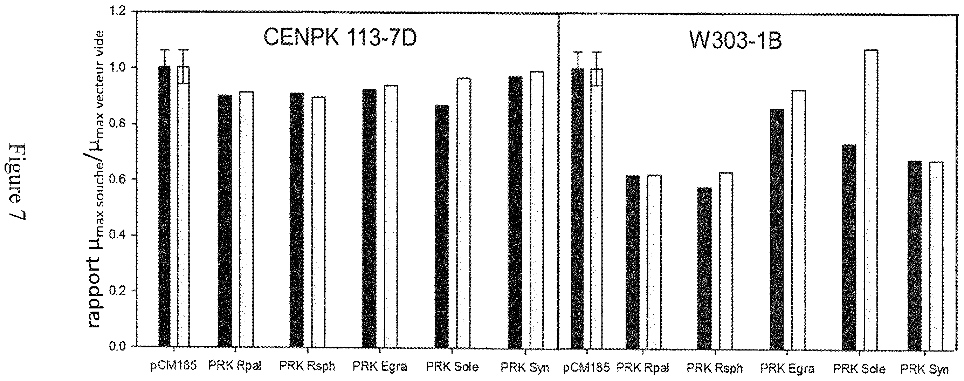

**Figure 7** : rapports de taux de croissance **µ** maximum (µₘₐₓ) pour chaque souche. Barres foncées : état induit (haut niveau expression) du promoteur tetO (milieu ne contenant pas de doxycycline) ; barres claires : état (partiellement) réprimé (bas niveau expression) du promoteur tetO (milieu contenant 2 µg/mL de doxycycline).

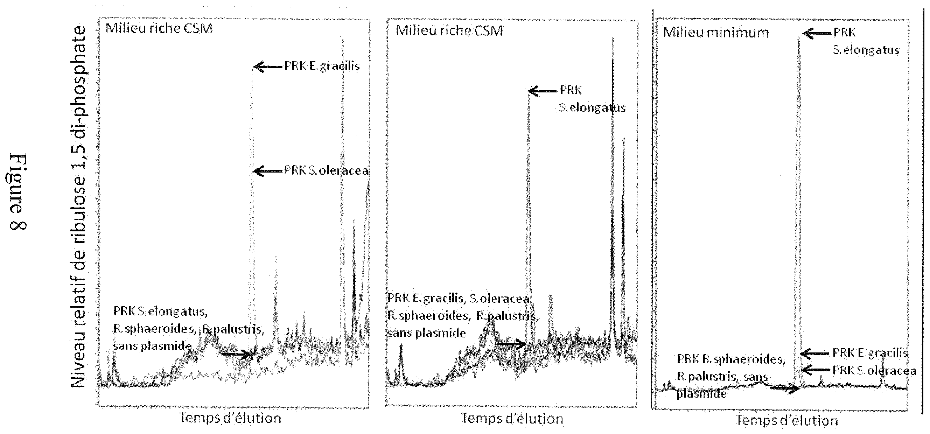

**Figure 8** : détection du ribulose 1,5-disphosphate (de masse molaire 309 g/mol) en fonction du temps d'élution dans les différents extraits. Le panneau de gauche montre les chromatogrammes de la souche W303-1B cultivée dans des tubes fermés contenant le milieu CSM sélectif ; le panneau du centre montre les chromatogrammes de la souche CENPK cultivée dans des tubes fermés et milieu de sélection (milieu CSM), le panneau de droite représente les chromatogrammes obtenus pour la souche CENPK cultivée dans des tubes fermés et milieu minimum.

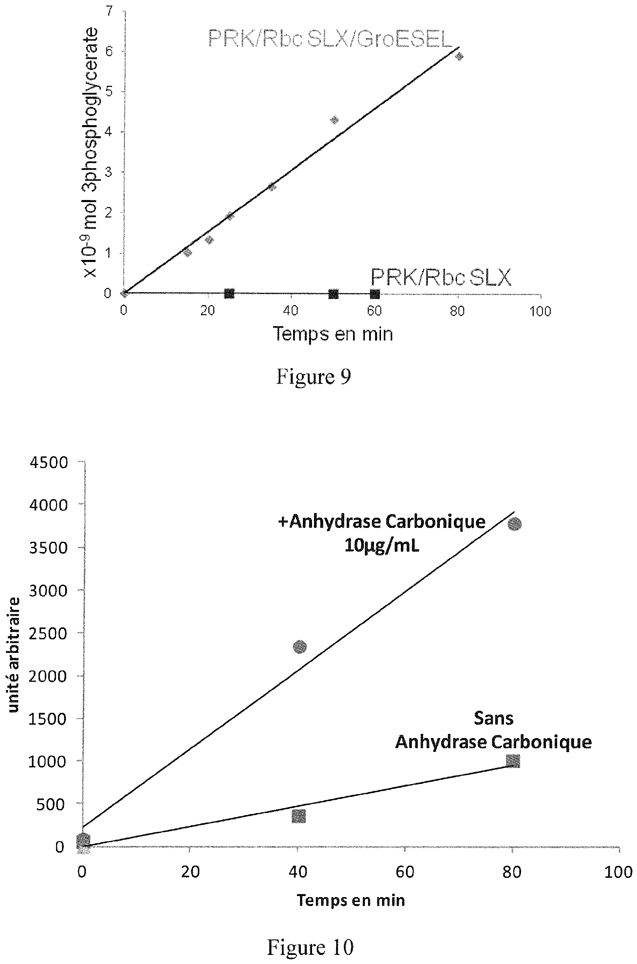

**Figure 9** : Evaluation de l'activité enzymatique du complexe RuBisCO synthétique sur les souches CEN.PK n°3 et 4. PRK : phosphoribilokinase ; RbcSLX : produits des gènes RbcL, RbcS et RBCX, GroESL : produits des gènes GroEL et GroEL de *E*. *coli.*
**Figure 10** : Activité RuBisCO, d'un extrait de levure CEN-PK n°3 contenant l'ingénierie complète, en présence et en absence d'anhydrase carbonique bovine.

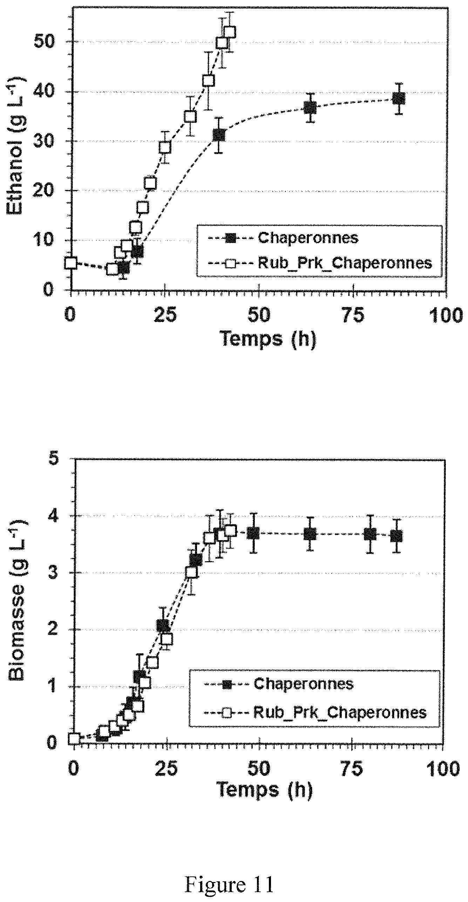

**Figure11** : Evolution de la concentration en éthanol et en biomasse lors de cultures anaérobies (Rub : RbcS+RbcL; Prk : phosphoribulokinase ; Chaperonnes : RbcX+(GroES+GroEL) E. coli). ■ souche CEN.PK n°13b et □ souche CEN.PK n°3.

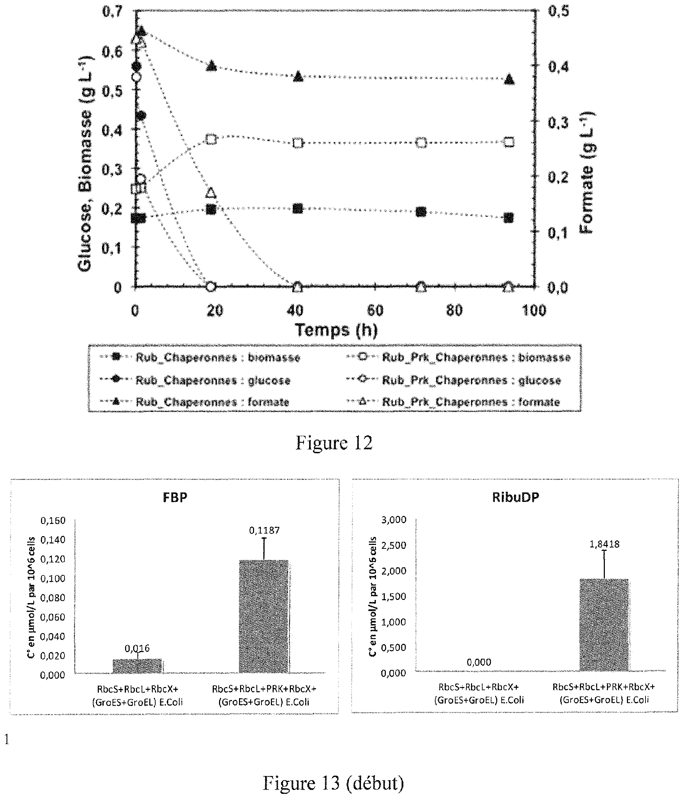

**Figure 12** : Evolution des concentrations en biomasse, formate et glucose lors de cultures aérobies (Rub : RbcS+RbcL; Prk : phosphoribulokinase ; Chaperonnes : RbcX+(GroES+GroEL) E. coli). Génération de biomasse (g.L⁻¹ ordonnée de gauche): ■ souche CEN.PK n°2 (RbcL, RbcS, RbcX, GroES-coli, GroEL_coli), □ souche CEN.PK n°3 (PRK, RbcL, RbcS, RbcX, GroES_coli, GroEL_coli); Consommation de glucose (g.L⁻¹ ordonnée de gauche): ● souche CEN.PK n°2, ○ souche CEN.PK n°3 ; Consommation de formate (g.L⁻¹ ordonnée de droite): ▲souche CEN.PK n°2, Δ souche CEN.PK n°3.

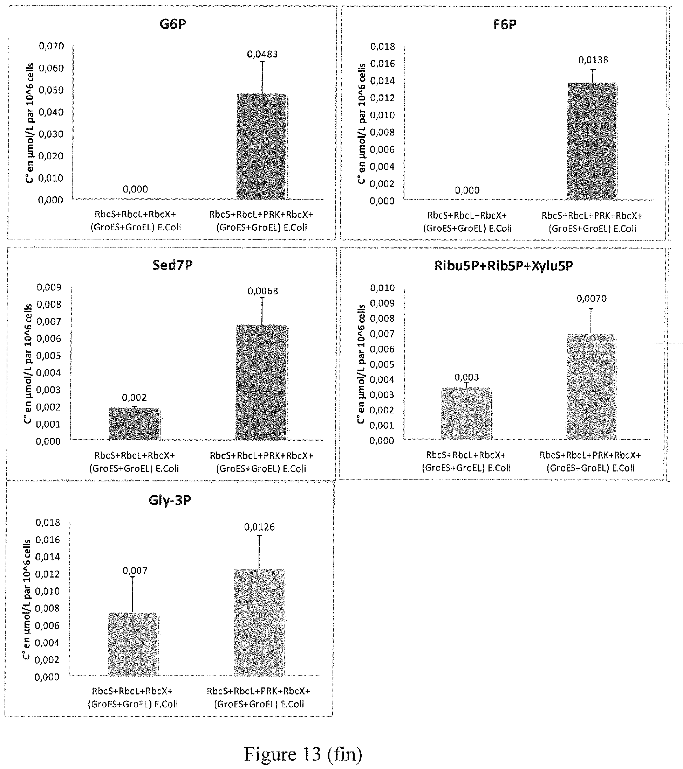

**Figure 13** : Extraits de l'analyse métabolomique faite sur les souches comportant l'ingénierie complète (CEN.PK n°3(PRK, RbcL, RbcS, RbcX, GroES_coli, GroEL-coli)) ou sans PRK (CEN.PK n°2(RbcL, RbcS, RbcX, GroES_coli, GroEL-coli)). L'analyse montre notamment une forte accumulation en Fructose-6P (F6P), Fructose-1,6diP (FBP), Glucose-6P (G6P), Sedoheptulose-7P (S7P), Xylolose-5P (Xylu5P), Ribulose-5P (Ribu5P), Ribose-5P (Rib5P), Ribulose-1,5diP (RibuDP) et Glycérate-3P (Gly3P).

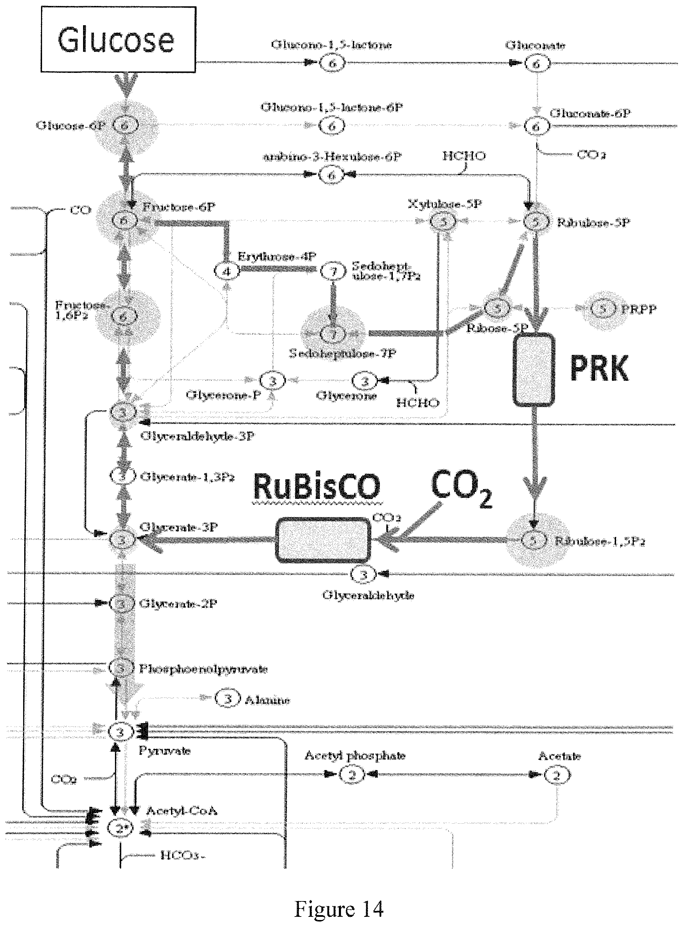

**Figure 14** : simulation métabolique pour une cellule de levure modifiée conformément à l'invention.

### EXEMPLE 1 : EXPRESSION ET ASSEMBLAGE DU COMPLEXE RUBISCO DE SYNECHOCCOCUS ELONGATUS CHEZ LA LEVURE SACCHAROMYCES CEREVISIAE.

Des gènes synthétiques codant pour les sous-unités RbcS et RbcL et pour la chaperonne spécifique RbcX de la RuBisCO de *Synechoccocus elongatus* pCC6301, et optimisés pour l'expression dans la levure, ont été préparés, et clonés dans le plasmide pBSII (Genecust). Des variants dans lesquels une étiquette HA a été ajoutée à l'extrémité 3' de la séquence codante ont également été construits.

Les séquences de ces gènes synthétiques (sans l'étiquette HA) sont indiquées dans la liste de séquences en annexe sous les numéros SEQ ID NO: 1 (RbcL), SEQ ID No : 2 (RbcS) et SEQ ID NO:3 (RbcX).

Les séquences codant pour les chaperonnes GroES et GroEL de *E. coli* ont été amplifiées à partir de cultures d'*E. coli* et clonées dans le plasmide pSC-B-amp/kan (Stratagène) .

Les séquences récupérées à partir des vecteurs de clonage ont été introduites dans des vecteurs d'expression de levure. Ces vecteurs hôtes sont listés dans le Tableau I ci-dessous.

**Tableau I**

| Noms | Origine réplication levure | Marqueur de sélection | Cassette de transcription (promoteur-terminateur) | Réplicon E. coli |
|---|---|---|---|---|
| pFPP5 | 2u | URA3 | pGAL10-CYC1 -tPGK | Yes (AmpR) |
| pFPP10 | 2u | URA3 | pTDH3- -tADH | Yes (AmpR) |
| pFPP11 | 2u | URA3 | pTDH3- -tCYC1 | Yes (AmpR) |
| pFPP12 | 2u | URA3 | pTGI1--tCYC1 | Yes (AmpR) |
| pFPP13 | ARS-CEN6 | LEU2 | pTEF1-tPGK | Yes (AmpR) |

| | | | | |
|---|---|---|---|---|
| Note : pGAL10-CYC1 : promoteur synthétique composé de l'UAS du gène GAL10 et de l'initiation de transcription du gène CYC1 (POMPON et al, Methods Enzymol, 272, 51-64, 1996). | | | | |

Les cassettes d'expression ainsi obtenues sont listées dans le Tableau II ci-dessous.

**Tableau II**

| Noms | Promoteur | Phase ouverte de lecture | Étiquette | Terminateur |
|---|---|---|---|---|
| CAS1 | TEF1 p | RbcL- | HA | PGK |
| CAS2 | TEF1p | RbcS- | HA | PGK |
| CAS3 | TEF1p | RbcX- | HA | PGK |
| CAS4 | PGI1p | RbcX | sans | CYC1 |
| CAS5 | TDH3p | RbcL- | HA | ADH1 |
| CAS6 | TDH3p | RbcL | sans | ADH1 |
| CAS16 | TEF1 | RbcS | Sans | PGK |
| CAS17 | TDH3 | RbcL- | HA | PGK |
| CAS18 | TDH3 | RbcL | Sans | ADH |
| CAS19 | TEF1p | RbcX | Sans | PGK |
| CAS20 | PGI1 | RbcX- | HA | CYC1 |
| CAS21 | PGI1p | GroES | Sans | CYC1 |
| CAS22 | TDH3 | GroEL | Sans | ADH |

Dans certains vecteurs, 2 ou 3 cassettes ont été insérées. Pour cela les plasmides ont été amplifiés dans la bactérie *Escherichia coli* DH5α et préparés par maxiprep, puis digérés par des enzymes de restriction adaptées. Enfin, les fragments sont intégrés aux vecteurs hôtes par ligation à la ligase T4 (FERMENTAS). La liste des vecteurs construits est indiquée dans le Tableau III ci-dessous.

**Tableau III**

| Noms | Type Origine | Cassette 1 | Cassette2 | Cassette 3 | marqueurs | Vecteur hôte |
|---|---|---|---|---|---|---|
| pFPP6 | 2u | CAS1 | sans | sans | URA3 | pFPP5 |
| pFPP7 | 2u | CAS2 | sans | sans | URA3 | pFPP5 |
| pFPP18 | 2u | CAS2* | CAS6 | sans | URA3 | pFPP5/pFPP10 |
| pFPP19 | 2u | CAS2 | CAS6 | sans | URA3 | pFPP5/pFPP10 |
| pFPP23 | ARS416-CEN6 | CAS3 | sans | sans | LEU2 | pFPP13 |
| pFPP40 | 2u | CAS5 | sans | sans | URA3 | pFPP10 |
| pFPP45 | 2u | CAS6 | CAS16 | sans | URA3 | pFPP5/pFPP10 |
| pFPP48 | 2u | CAS20 | sans | sans | URA3 | pFPP12 |
| pFPP49 | 2u | CAS19 | sans | sans | LEU2 | pFPP12/pFPP13 |
| pFPP55 | ARS415-CEN6 | CAS19 | CAS21 | CAS22 | LEU2 | pFPP13 |
| pFPP56 | ARS415-CEN6 | CAS19 | CAS21 | CAS22* | LEU2 | pFPP13 |

| | | | | | | |
|---|---|---|---|---|---|---|
| * orientation Inverse | | | | | | |

Différents vecteurs ou combinaisons de vecteurs ont été utilisés pour transformer des cellules de levure *S. cerevisiae* (souche W303.1B).

Ces vecteurs et combinaisons de vecteurs sont indiqués dans le Tableau IV ci-dessous.

**Tableau IV**

| Souche transformée | Souche parente | Vecteur 1 | Vecteur 2 | Vecteur 3 | Protéines exprimées (§ indique une fusion C-terminale avec une étiquette HA) | | | | | |
|---|---|---|---|---|---|---|---|---|---|---|
| | | | | | RbcS | RbcL | RbcX | GroES | GroEL | PRK |
| 11.19 | W303 | pCM185 | pFPP23 | pFPP19 | X§ | X | X§ | | | |
| 18.3 | W303 | pFPP45 | pFPP49 | | X | X | X | | | |
| 22.2 | W303 | pFPP45 | pFPP56 | | X | X | X | X | X | |
| 22.3 | W303 | pFPP45 | pFPP55 | | X | X | X | X | X | |
| 30.1 | W303 | pCM185 | | | | | | | | |
| 11.5 | W303 | pCM185 | pFL36 | pFPP5 | | | | | | |
| 11.7 | W303 | pCM185 | pFL36 | pFPP18 | X§ | X | | | | |
| 11.9 | W303 | pCM185 | pFL36 | pFPP19 | X§ | X | | | | |
| 11.15 | W303 | pCM185 | pFPP23 | pFPP5 | | | X§ | | | |
| 11.17 | W303 | pCM185 | pFPP23 | pFPP18 | X§ | X | X§ | | | |
| 14.5 | W303 | pFPP6 | | | | X§ | | | | |
| 14.12 | W303 | pFPP40 | | | | X§ | | | | |
| 14.6 | W303 | pFPP7 | | | X§ | | | | | |
| 14.7 | W303 | pFPP23 | | | | | X§ | | | |
| 16.3 | W303 | pFPP48 | | | | | X§ | | | |
| 16.5 | W303 | pFPP43 | pFPP23 | | X§ | X§ | X§ | | | |
| 16.6 | W303 | pFPP43 | | | X§ | X§ | | | | |
| yFB3 | CEN.PK | pFPP45 | pFPP20 | pFPP56 | X | X | X | X | X | X |

| | | | | | | | | | | |
|---|---|---|---|---|---|---|---|---|---|---|
| Notes : pCM185 : plasmide ATCC 87659 ; pFL36 : plasmide ATCC 77202 | | | | | | | | | | |

Les cellules transformées sont cultivées à 30°C à l'air ambiant sur milieu YNB (yeast without nitrogen base supplémenté en sulfate d'ammonium 6,7 g.L⁻¹, glucose 20 g.L⁻¹, agar 20 g.L⁻¹ pour les géloses) complémenté avec un milieu commercial CSM (MP Biomedicals) adapté aux marqueurs de sélection des plasmides utilisés pour la transformation. Les cultures sont arrêtées par refroidissement à 4°C une génération avant la fin de la phase exponentielle.

Un aliquot est prélevé sur chaque culture et les cellules sont lysées à la soude en présence de SDS pour l'analyse des protéines totales sur gel SDS dénaturant.

Le reste des cultures est centrifugé, puis des sphéroplastes sont préparés par digestion enzymatique des parois cellulaires avec un mélange zymolyase-cytohélicase en milieu sorbitol hypertonique (1,2M sorbitol). Les sphéroplases sont lavés en milieu hypertonique sorbitol en présence de concentrations saturantes de PMSF et d'EDTA (inhibiteurs des protéases), puis cassés par pipetage répétés et sonication légère en milieu isotonique (0,6M) sorbitol. Après centrifugation à basse vitesse (1500 rpm) pour éliminer les gros débris puis à moyenne vitesse (4000rpm) pour récupérer les débris de tailles intermédiaires et les mitochondries, le surnageant est récupéré et les protéines sont précipitées à 80 % de saturation de sulfate d'ammonium avec maintien du pH à 6,5-7,0. Le précipité est redissous et dialysé en présence d'inhibiteurs des protéases, puis fractionné par tamisage moléculaire sur une colonne de Sephacryl S300 (GE Healthcare). Les fractions éluées sont regroupées par pools pour l'analyse sur gel.

Le lysat total et les fractions triées par poids moléculaires (gamme protéine globulaire native de 10⁴ à 1.5x 10⁶ daltons) sont analysées sur gel dénaturant SDS-PAGE et sur gel non-dénaturant (précoloration au bleu de coomassie-PAGE). Les gels sont colorés au bleu de Coomassie et le blot au rouge Ponceau pour l'analyse des protéines totales. Les protéines RbcL RbcS et RbcX sont détectées après électro-transfert sur nylon chargé par immuno-détection. Dans le cas de RbcL la détection peut s'effectuer directement à l'aide d'un anticorps anti-RbcL, et dans le cas de RbcS et RbcX, indirectement par l'intermédiaire d'un anticorps anti étiquette HA. Les différentes expériences ont été répétées en alternant la coexpression de protéines étiquetées ou non afin de vérifier que la présence des étiquettes n'affectait pas le repliement ou l'assemblage des complexes.

La Figure 1 représente l'analyse de lysats totaux de souches transformées.

Les 2 sous-unités sont exprimées dans la levure. RbcL est exprimée à haut niveau (visible en coloration non spécifique des protéines totales d'un extrait). Le niveau d'expression de RbcS n'a pas été quantifié mais semble similaire à celui de RbcL sur la base des immunodétections anti-HA. Les deux protéines ne présentent aucun signe de dégradation (absence de bandes floues ou multiples) suggérant une bonne qualité de repliement et une résistance aux protéases endogènes. La chaperone RbcX est aussi bien exprimée et ne présente aucun signe de dégradation. Les systèmes plasmidiques de coexpression des 3 composants sont opérationnels et ne démontrent pas d'interférence notable entre l'expression des différents composants.

La Figure 2 représente l'analyse par immunodétection d'un lysat total (encadré sur la gauche de la Figure), et des fractions triées par poids moléculaire, de la souche 16.5, qui co-exprime RbcL, RbcS, et RbcX et de son contrôle, la souche 16.3 qui exprime RbcX.

On observe une distribution mono-modale de la sous unité RbCL au sein de complexes de taille supérieure ou égale à 500 kD alors que la masse de la sous-unité isolée est de 55 kD. La distribution de RbcS et RbcX est au contraire bimodale, un mode étant de taille similaire à celle observée pour RbcL, l'autre correspondant à des petites tailles, proches de celles des protéines RbcS et RbcX isolées. Le complexe RuBisCO natif n'est pas visible de manière probante en gel natif et en coloration non spécifique à la taille attendue (environ 500 kD) dans ces conditions. Néanmoins un complexe de très large taille est détectable au environ de 750-1000 kD (plus grand que la taille prévue) par immunodétection de RbcL.

La Figure 3 représente les résultats de l'analyse sur gel non-dénaturant, suivie d'immunodétection à l'aide d'un anticorps anti-RbcL, des extraits totaux des souches 11.9, 18.3 et 22.2, et de fractions triées par poids moléculaire de la souche 22.2, qui co-exprime RbcL, RbcS, et RbcX, de *S. Elongatus* et les chaperonnes de *E. coli.* Puis en parallèle les fractions triées par poids moléculaire des souches 18.3 (à gauche) et 22.2 (à droite).

Ces résultats montrent que la co-expression avec les chaperonnes GroES et GroEL induit une réduction de la taille du complexe de haut poids moléculaire (environ de 750-1000 kD) qui était détecté en l'absence de ces chaperonnes ; on observe dans les cellules co-exprimant RbcL, RbcS, RbcX, GroES et GroEL, une bande bien définie correspondant à la taille attendue (environ 500kD) pour le complexe RuBisCO natif.

Ces résultats montrent qu'un complexe RuBisCO de forme I d'origine procaryote peut être exprimé et s'assembler correctement dans des cellules de *S. cerevisiae,* cet assemblage étant amélioré par la présence de chaperonnes généralistes GroES et GroEL.

Pour l'analyse de l'activité RuBisCO *in vitro* l'extraction des protéines solubles de la souche yFB3 est réalisée. Les cellules sont cultivées à 30°C à l'air ambiant sur milieu YNB (yeast without nitrogen base), supplémenté en sulfate d'ammonium 6,7 g.L⁻¹, glucose 20 g.L⁻¹, agar 20 g.L⁻¹ pour les géloses) avec un milieu commercial CSM (MP Biomedicals), et adapté aux marqueurs de sélection des plasmides utilisés (milieu sans Leucine, sans Uracile et sans Tryptophane pour yFB3). Les cultures sont arrêtées par refroidissement à 4°C une génération avant la fin de la phase exponentielle. Les cultures sont centrifugées, puis des sphéroplastes sont préparés par digestion enzymatique des parois cellulaires avec un mélange zymolyase-cytohélicase en milieu sorbitol hypertonique (1,2M sorbitol). Les sphéroplastes sont lavés en milieu hypertonique sorbitol en présence de 1 mM de PMSF et d'EDTA (inhibiteurs des protéases), puis cassés par pipetages répétés et sonication légère en milieu isotonique (0,6M) sorbitol. Après centrifugation à basse vitesse (200 g pendant 5 min) pour éliminer les gros débris puis à moyenne vitesse (1500 g pendant 10 min) pour récupérer les débris de tailles intermédiaires et les mitochondries, le surnageant est récupéré.

Les tests d'activité sur les extraits protéiques sont réalisés dans 50mM TRIS/HCl (pH7.5), 60mM NaHC03 (¹³C ou ¹²C) 10 mM MgC12 en présence de 2 mM de ribulose diphosphate (RiDP) et de 0.5 mg/ml de protéines totales d'extraits de yFB3. A t=10 min et t=60 min, 100 pL du mélange réactionnel sont prélevés, la réaction est arrêtée par l'ajout de 2 µl d'HCl, et le prélèvement est centrifugé 10 min à 9300 g puis analysé par HPLM/MS (Phase réverse C18 en appariement d'ions avec un gradient tributylamine acétate10mM /acétonitrile pH 6.0). Les métabolites sont détectés par spectrométrie de masse électrospray en ionisation négative, et identifiés sur la base de leurs rapports m/e et de leurs temps d'élution, comparés à ceux de composés standards.

Les résultats sont illustrés par la Figure 4.

En présence de ¹³CO₂, le taux de marquage du 3-phosphoglycérate formé à 60 min est de 52% comme attendu. En effet, comme représenté en bas de la figure 4, la réaction catalysée par la RuBisCO est la formation de 2 molécules de 3-phosphoglycérate à partir d'une molécule de CO₂ et d'une molécule de RiDP. En présence de ¹²CO₂, le 3-phosphoglycérate est formé mais uniquement sous sa forme non marquée.

La RuBisCO présente dans les extraits est donc capable d'incorporer le carbone du CO₂ pour produire du 3-phosphoglycérate.

### EXEMPLE 2 : EXPRESSION DE PHOSPHORIBULOKINASES CHEZ LA LEVURE SACCHAROMYCES CEREVISIAE.

Des gènes synthétiques codant pour 5 PRKs d'origine différente : *Synechococcus elongatus* (Syn), *Rhodobacter sphaeroides* (Rsph), *Rhodopseudomonas palustris* (Rpal), *Spinnacia oleracea* (Sole), *Euglena gracilis* (Egra) et optimisés pour l'expression dans la levure, et flanqués ou non d'une étiquette HA en C-terminal, ont été préparés. Les séquences de ces gènes synthétiques (sans l'étiquette HA) sont respectivement indiquées dans la liste de séquences en annexe sous les numéros SEQ ID NO: 4 à SEQ ID NO: 8.

Les PRKs Rsph et Rpal sont des PRKs de classe I existant sous forme native en octamère pour Rsph et hexamère pour Rpa. Les kinases Sole, Egra et Syn sont des kinases de classe II dont la forme native est un dimère pour les deux premières, et un tétramère pour la troisième.

Les séquences de *Rhodobacter sphaeroïdes (Rsph), Rhodopseudomonas palustris (Rpal), Euglena gracilis (Egra) et ,Spinacia oleracea (Sole)* ont été synthétisées par la société Genecust et livrées dans un plasmide pBlueScript II+. Les plasmides ont été amplifiés dans la bactérie *Escherichia coli* DH5α. Une extraction par maxiprep est réalisée pour chacun des plasmides. Ils sont ensuite digérés par les enzymes *BamHI* et *PstI* puis le produit de la digestion est déposé sur un gel d'agarose 0,8% contenant du SYBER Safe. La migration est réalisée dans du tampon TAE 1X à 50V pendant 30 minutes. Les bandes correspondantes aux phases ouvertes de lecture (972 bp pour PRK de *Rpal*, 966 bp pour *Rsph*, 1461 bp pour *Egra* et 1277 pour *Sole*) sont découpées dans le gel et l'ADN est extrait avec le kit d'extraction pour gel de la société FERMENTAS. Enfin, les fragments sont intégrés aux vecteurs pCM185, pCM188-2 et pCM188-7 par ligation à la ligase T4 (FERMENTAS) sous contrôle du promoteur tetO répressible par la doxycycline, pour donner respectivement les vecteurs d'expression pFPP20, pJLP1, pJLP2, pJLP3, pJLP4.

Les cassettes et vecteurs d'expression ainsi obtenus sont listés dans le Tableau V ci-dessous.

**Tableau V**

| Noms | Type Origine | Promoteur | Terminateur | Phase ouverte de lecture | Marqueurs | Vecteur hôte |
|---|---|---|---|---|---|---|
| pCM185 | ARS416-CEN4 | TetO7 | CYC1 | Ø | TRP1 | Ø |
| pCM188-2 | ARS416-CEN4 | TetO7 | CYC1 | Ø | URA3 | Ø |
| pCM188-7 | ARS416-CEN4 | TetO7 | CYC1 | Ø | URA3 | Ø |
| pFPP20 | ARS416-CEN4 | TetO7 | CYC1 | PRK *S. elongatus* | TRP1 | pCM185 |
| pFPP21 | ARS416-CEN4 | TetO2 | CYC1 | PRK *S. elongatus* | URA3 | pCM188-2 |
| pFPP22 | ARS416-CEN4 | TetO7 | CYC1 | PRK *S. elongatus* | URA3 | pCM188-7 |
| pJLP1 | ARS416-CEN4 | TetO7 | CYC1 | PRK *E. gracilis* tag HA | TRP1 | pCM185 |
| pJLP2 | ARS416-CEN4 | TetO7 | CYC1 | PRK *R. sphaeroides* tag HA | TRP1 | pCM185 |
| pJLP3 | ARS416-CEN4 | TetO7 | CYC1 | PRK *R. palustris* tag HA | TRP1 | pCM185 |
| pJLP4 | ARS416-CEN4 | TetO7 | CYC1 | PRK *S. oleracea* tag HA | TRP1 | pCM185 |

Ces vecteurs ont été utilisés pour transformer des cellules de *S. cerevisiae* des souches W303.1B et CNPK. La première de ces souches est une souche typique de laboratoire, la seconde est une souche semi-industrielle.

La transformation a été effectuée selon le protocole de CHEN et al (Curr Genet. 1992, 21, 83-4), en maintenant à toutes les étapes de transformation et de sous clonage une concentration de doxycycline de 2 pg/ml, adaptée à la répression du promoteur tetO. Les transformants ont été stockés en milieu glycérolé (50% gycerol) à -80C en présence de 2 pg/ml doxycycline.

Les souches transformées obtenues sont listées dans le Tableau VI ci-dessous.

**Tableau VI**

| Souche transformée | Souche parente | Vecteur 1 | Vecteur 2 | Vecteur 3 | PRK exprimée |
|---|---|---|---|---|---|
| 11.5 | W303 | pCM185 | pFL36 | pFPP5 | |
| 30.2 | W303 | pFPP20 | | | PRKsvn |
| 11.6 | W303 | pFPP20 | pFL36 | pFPP5 | PRKsvn |
| yJL1 | W303 | pJLP1 | | | PRK Egra |
| yJL2 | W303 | pJLP2 | | | PRK Rsph |
| yJL3 | W303 | pJLP3 | | | PRK Rpal |
| yJL4 | W303 | pJLP4 | | | PRK Sole |
| yJL5 | CENPK | pFPP20 | | | PRKsvn |
| yJL6 | CENPK | pJLP1 | | | PRK Egra |
| yJL7 | CENPK | pJLP2 | | | PRK Rsph |
| yJL8 | CENPK | pJLP3 | | | PRK Rpal |
| yJL9 | CENPK | pJLP4 | | | PRK Sole |

Les cellules transformées sont mises en préculture à partir du stock dans un milieu YNB (yeast without nitrogen base supplémenté en sulfate d'ammonium 6,7 g.L⁻¹, glucose 20 g.L⁻¹, agar 20 g.L⁻¹ pour les géloses) avec un milieu commercial CSM (MP Biomedicalsadaptés au maintien de la sélection du plasmide et contenant une concentration de 2 µg/*ml* doxycycline adaptée à la répression de l'expression de la PRK.

L'influence de l'expression des différentes PRKs sur la viabilité des cellules a été évaluée sur milieu gélosé en présence ou non de doxycycline : Chaque souche est cultivée en liquide sur milieu CSM sélectif avec doxycycline à 2 µg/mL. Un équivalent de 2 DO (DO à 600nm) est récupéré puis lavé 2 fois pour éliminer la doxycycline. Des dilutions au dixième sont réalisées. 10µL des dilutions sont déposées sous forme de gouttes (séries de dilutions sérielles) des suspensions de cellules, sur des boites de milieux gélosés (contenant ou non de la 2 µg/mL doxycycline) et incubées à 28°C en atmosphère normale ou alternativement en sac fermés dont l'atmosphère contient au moins 90 :10 de dioxyde de carbone/air (vol/vol).

Les résultats en atmosphère normale sont illustrés par la Figure 5. Les résultats en atmosphère riche en CO₂ sont illustrés par la Figure 6.

On constate que toutes les PRKs sont plus ou moins toxiques dans la souche W3031B à haut niveau d'expression (induites). Néanmoins la toxicité apparait beaucoup plus faible dans la souche CENPK, ou seule la PRK de Syn est toxique à l'état induit.

D'autres expériences montrent que dans la souche W303.1B, la toxicité est fortement atténuée dans une atmosphère pauvre en oxygène et riche en dioxyde de carbone.

L'influence de l'expression des différentes PRKs sur la croissance des cellules a été évaluée sur des cultures en milieu liquide : Les souches sont cultivées en milieu sélectif CSM dans des tubes fermés (contenant ou non de la 2 µg/mL doxycycline). La croissance est suivie grâce à la mesure de la densité optique à 600 nm jusqu'à l'entrée en phase stationnaire. Pour chaque souche on réalise le rapport entre le taux de croissance µ (accroissement de la population par unité de temps) maximum de la souche et celui de la souche témoin (souche + plasmide vide).

Les rapports de taux de croissance **µ** maximum (µₘₐₓ) pour chaque souche sont illustrés par la Figure 7.

Ces résultats confirment la toxicité moindre des kinases dans le contexte de la souche CNPK 113-7D que dans celui de la souche W303.1B.

Un effet de toxicité dose (niveau d'induction)-réponse (taux de croissance) est observé uniquement pour la kinase Sole et chez W303.1B.

Chez W303.1B, on observe une toxicité significative des kinases Rpal, Rsph, Syn faiblement et fortement exprimées. La toxicité apparaît plus faible pour la kinase Egra.

Pour l'analyse du métabolite Ribulose-1,5 bisphosphate du cycle carboné central, on effectue le lavage des cellules pour éliminer la doxycycline et leur mise en culture liquide à 30°C sur milieu YNB (yeast without nitrogen base) supplémenté en sulfate d'ammonium 6,7 g.L⁻¹, glucose 20 g.L⁻¹, (agar 20 g.L⁻¹ pour les géloses) complémenté avec un milieu commercial CSM (MP Biomedicals), adapté, selon le marqueur de sélection du plasmide utilisé. Les cultures sont effectuées en tube fermé sans apport d'oxygène au-delà de 3-10 volumes d'air (non renouvelé) par volume de milieu de culture. Le dioxyde de carbone résultant de la culture est ainsi maintenu dans le volume du tube de culture. Cette procédure permet de limiter la toxicité de l'expression.

Le métabolisme est bloqué par dilution de la culture dans du méthanol-eau 60:40 (v/v) à -80°c (mélange maintenu à - 40°C dans un bain carboglace/acétonitrile), suivi de centrifugation rapide (température maintenue inférieure à -20°C) et de lyse des cellules dans un mélange méthanol-eau (60 :40 v/v) contenant de la soude 0.3M puis de congélation à -80°C selon le protocole décrit par LUO et al., (J. Chromatography A 1147:153-164, 2007).

Après décongélation un aliquot est neutralisé par de l'acide acétique glacial, centrifugé et le surnageant analysé par HPLM/MS (Phase réverse C18 en appariement d'ions avec un gradient tributylamine acetate/ acetonitrile pH 6.0). Les métabolites sont détectés par spectrométrie de masse électrospray en ionisation négative, et identifiés sur la base de leurs rapports de masse m/e et de leurs temps d'élution, comparés à ceux de composés standards.

Les résultats sont illustrés par la Figure 8.

Le niveau d'activité (non normalisé pour le niveau d'expression) estimé par le niveau d'accumulation du ribulose 1,5 diphosphate produit de la réaction apparait :
- très élevé pour la PRK Syn même en condition réprimée (50% du niveau induit). Cette activité s'accompagne d'une toxicité avec chute importante du niveau d'ATP intracellulaire ;
- détectable mais plus faible pour les PRKs Egra et Sole en milieu minimum ;
- indétectable dans les conditions utilisées pour les PRKs RspH et Rpai ;
- dépendant du milieu de culture, avec pour la PRK Syn un niveau d'accumulation de ribulose 1,5 diphosphate beaucoup plus élevé en milieu pauvre que riche.

L'ensemble de ces observations indique que seules les kinases de classe II conduisent à l'accumulation de niveaux élevés de ribulose diphosphate chez S. cerevisiae.

### EXEMPLE 3 : CARACTERISATION PHENOTYPIQUE DES SOUCHES CONTENANT L'INGENIERIE "CARBOYEAST" IN VITRO PAR L'ETUDE DE LA FONCTIONNALITE DU COMPLEXE RUBISCO EXPRIME CHEZ LA LEVURE ET DES PARAMETRES LA CONTROLANT

Comme décrit dans la figure 4, la fonctionnalité du complexe RuBisCO artificiel a été mise en évidence *in vitro* par des tests d'activité RuBisCO sur un substrat synthétique (Ribulose diphosphate) à partir d'extraits de levure contenant l'ingénierie complète ou partielle, et en évaluant l'apparition du produit de la réaction qu'elle catalyse, à savoir le 3-glycérophosphate.

### 3.1. Constructions et souches utilisées

Le présent exemple a été réalisé en utilisant les constructions et les souches transformées décrites dans les Tableaux VII à IX ci-dessous.

**Tableau VII. Cassettes d'expression**

| Noms | Promoteur | Phase ouverte de lecture | Etiquette | Terminateur |
|---|---|---|---|---|
| CAS6 | TDH3p | RbcL S. elongatus optimisée | sans | ADH1t |
| CAS7 | TetO7p | PRK S. elongatus optimisée | sans | CYC1t |
| CAS16 | TEF1p | RbcS S. elongatus optimisée | sans | PGKt |
| CAS19 | TEF1p | RbcX S. elongatus optimisée | Sans | PGKt |
| CAS21 | PGI1p | GroES E. coli | Sans | CYC1t |
| CAS22 | TDH3p | GroEL E. Coli | Sans | ADH1t |
| CAS23 | PGI1p | GroES S. elongatus optimisée | sans | CYC1t |
| CAS25 | TDH3p | GroEL2 S. elongatus optimisée | sans | ADH1t |
| CAS28 | PGI1p | polylinker | sans | CYC1t |
| CAS33 | TEF1p | polylinker | Sans | PGKt |

**Tableau VIII. Vecteurs d'expression (Références à Tableau VII pour les cassettes)**

| Noms | Type Origine | Cassette 1 | Cassette 2 | Cassette 3 | Marqueurs d'auxotrophie | Vecteur Hôte | Réplicon E. Coli |
|---|---|---|---|---|---|---|---|
| pFPP13 | ARS415-CEN6 | CAS33 | sans | sans | LEU2 | pFL36 | Oui (AmpR) |
| pFFP53 | ARS415-CEN6 | CAS19 | CAS28 | sans | LEU2 | pFL36 | Oui (AmpR) |
| pFFP56 | ARS415-CEN6 | CAS19 | CAS21 | CAS22* | LEU2 | pFL36 | Oui (AmpR) |
| pFB05 | ARS415-CEN6 | CAS19 | CAS25* | CAS21 | LEU2 | pFFP56 | Oui (AmpR) |
| pFB07 | ARS415-CEN6 | CAS23 | CAS22* | CAS19 | LEU2 | pFFP56 | Oui (AmpR) |
| pFB08 | ARS415-CEN6 | CAS23 | CAS25* | CAS19 | LEU2 | pFFP56 | Oui (AmpR) |
| pFB09 | ARS415-CEN6 | CAS21 | CAS22* | sans | LEU2 | pFFP56 | Oui (AmpR) |
| pFPP45 | 2µ | CAS6 | CAS16 | sans | URA3 | PYeDP51 | Oui (AmpR) |
| pFPP20 | ARS416-CEN4 | CAS7 | sans | sans | TRP | pCM185 | Oui (AmpR) |

| | | | | | | | |
|---|---|---|---|---|---|---|---|
| * orientation Inverse | | | | | | | |

**Tableau IX. Combinaison de plasmides et souches (références à Tableau VIII.)**

| N°combinaiso n | Souche parente | Vecteur 1 | Vecteur 2 | Vecteu r 3 | Protéines exprimées | | | | | |
|---|---|---|---|---|---|---|---|---|---|---|
| | | | | | RbcS | RbcL | RbcX | PRKsyn | GroES | GroEL |
| 1b | CEN.PK 1605 | pYeDP51 | pCM185 | pFPP13 | | | | | | |
| 2 | CEN.PK 1605 | pFPP45 | pCM185 | pFPP56 | X | X | X | | coli | coli |
| 3 | CEN.PK 1605 | pFPP45 | pFPP20 | pFPP56 | X | X | X | syn | coli | coli |
| 4 | CEN.PK 1605 | pFPP45 | pFPP20 | pFPP53 | X | X | X | syn | | |
| 5 | CEN.PK 1605 | pFPP45 | pCM185 | pFPP53 | X | X | X | | | |
| 13b | CEN.PK 1605 | PYeDP51 | pCM185 | pFPP56 | | | X | | coli | coli |
| 15 | CEN.PK 1605 | PYeDP51 | pFPP20 | pFPP56 | | | X | syn | coli | coli |
| 17b | CEN.PK 1605 | pFPP45 | pFPP20 | pFPP13 | X | X | | syn | | |
| 101 | CEN.PK 1605 | pFPP45 | pFPP20 | pFB08 | X | X | X | Syn | syn | L2 syn |

| | | | | | | | | | | |
|---|---|---|---|---|---|---|---|---|---|---|
| (Syn : *S.elongatus* ; coli : *E.coli* ; L2 syn : GroEL2 *S*. *elongatus*) | | | | | | | | | | |

### Notes :

1. pCM185 : plasmide commercial (ATCC 87659)
2.pFL36 : Plasmide commercial (ATCC 77202)
3. PYeDP51 : Plasmide « vide », décrit dans l'article suivant : Urban P, Mignotte C, Kazmaier M, Delorme F, Pompon D. Cloning, yeast expression, and characterization of the coupling of two distantly related Arabidopsis thaliana NADPH-cytochrome P450 reductases with P450 CYP73A5. J Biol Chem. 1997 Aug 1;272(31):19176-86.
4. Les autres abréviations se rapportent aux gènes de *S. cerevisiae* décrits dans les banques de donnés.
5. Gènes synthétiques : Les gènes de *Synechoccocus elongatus* codant pour la chaperonne spécifique de l'assemblage de la RuBisCO (RbcX), ainsi que les chaperonnes généralistes GroES, GroEL1 et GroEL2 ont été resynthétisées après recodage pour la levure mettant en oeuvre un biais de codon inhomogène propriétaire et clonés dans pCC6301 (commercial).
6. Les chaperonnes de *E. coli* GroES et GroEL ont été amplifiées à partir de la bactérie, clonées dans pSC-B-amp/kan (Statagène) et montées sans recodage dans les vecteurs d'expression (cf. Exemple 1).
7. Les séquences de RbcS, RbcL, RbcX et PRK de *Synechoccocus elongatus* ont été décrites dans les Exemples 1 et 2.
8. Les séquences recodées des ADNc codant pour les chaperonines de *Synechoccocus elongatus* sont décrites dans le listage de séquences (SEQ ID No : 9 à 11) et insérées par recombinaison homologue dans le vecteur pUC57 préalablement linéarisé en cotransformant les deux molécules dans la levure. De la même manière, les ORFs ont été amplifiées par PCR à partir des constructions précédentes, générant des régions flanquantes homologues aux promoteurs et terminateurs portés par le vecteur pFPP56. Cela a permis un clonage par recombinaison homologue en co-transformant ce produit PCR dans une souche de levure avec le vecteur pFPP56 préalablement linéarisé, générant les différents vecteurs d'expression décrits tableau VIII selon les cassettes décrites tableau VII.

### 3.2. Evaluation de l'activité enzymatique du complexe RuBisCO synthétique

Pour l'extraction des protéines solubles des souches CEN.PK n°3 et CEN-PK n°4, les cellules sont cultivées à 30°C à l'air ambiant avec agitation sur milieu YNB (*yeast without nitrogen base*), supplémenté en sulfate d'ammonium 6,7 g. L⁻¹, glucose 20 g.L⁻¹, agar 20 g.L⁻¹ pour les géloses, avec un milieu commercial CSM (MP Biomedicals), adapté aux marqueurs de sélection des plasmides utilisés (milieu sans leucine, sans uracile et sans tryptophane). Les cultures sont arrêtées par refroidissement à 4°C une génération avant la fin de la phase exponentielle. Les cultures sont centrifugées, puis des sphéroplastes sont préparés par digestion enzymatique des parois cellulaires avec un mélange zymolyase-cytohélicase en milieu sorbitol hypertonique (1,2M sorbitol). Les sphéroplastes sont lavés en milieu hypertonique sorbitol en présence de 1 mM de PMSF et d'EDTA (inhibiteurs des protéases), puis cassés par pipetages répétés et sonication légère en milieu isotonique (0,6M sorbitol). Après centrifugation à basse vitesse (200 g pendant 5 min) pour éliminer les gros débris puis à moyenne vitesse (1500 g pendant 10min) pour récupérer les débris de tailles intermédiaires et les mitochondries, le surnageant est récupéré.

Les tests d'activité sur les extraits protéiques sont réalisés dans 50mM TRIS/HCl (pH7.5), 60mM NaHCO₃, 10 mM MgCl₂ en présence de 2 mM de ribulose diphosphate (RiDP) et de 0.05 mg/ml de protéines totales d'extraits. A différents temps, 60pL du mélange réactionnel sont prélevés, la réaction est arrêtée par l'ajout de 2µl d'HCl (12,1M), et le prélèvement est centrifugé 10 min à 9300 g puis analysé par HPLM/MS (Phase réverse C18 en appariement d'ions avec un gradient tributylamine acétate10mM /acétonitrile pH 6.0). Les métabolites sont détectés par spectrométrie de masse électrospray en ionisation négative, et identifiés sur la base de leurs rapports m/e et de leurs temps d'élution, comparés à ceux de composés standards.

Les résultats sont illustrés par la Figure 9. Cette Figure représente en ordonnée la quantité de moles de 3-phosphoglycérate détectées (m/e de 185) obtenue à différents temps de réaction en abscisse.

On note que pour une ingénierie complète (CEN.PK n°3 : RbcS+RbcL+PRK+chaperonnes RbcX et GroES et GroEL de *E*. *coli*), dite ingénierie "CARBOYEAST", et un substrat non limitant, la quantité du produit issu de l'activité catalytique de l'enzyme RuBisCO synthétique augmente linéairement dans le temps. Le complexe RuBisCO exprimé dans la levure par l'ingénierie est donc fonctionnel et stable.

Il apparait clairement que l'association du couple de chaperonnes bactériennes généralistes GroES GroEL à la chaperonne RbcX, spécialisée dans le repliement du complexe RuBisCO, est essentielle (Figure 9 et Tableau X).

Toutefois, dans les conditions du test illustrées, la co-expression d'une combinaison de chaperonnes bactériennes généralistes (GroES et GroEL), issue de *E. coli,* associée à la chaperonne spécifique RbcX, issue de *S. elongatus,* est plus efficace pour reconstruire la fonctionnalité du complexe RuBisCO, issu lui-même de *S. elongatus,* que la même association mais dont tous les acteurs sont issus du même organisme *S*. *elongatus* (Tableau X, lignes 1 et 3).

**Tableau X : Tests d'activité in vitro de la RuBisCO réalisés selon un protocole similaire à celui décrit précédemment à partir d'extraits de souches CEN-PK cultivées sur glucose et contenant l'ingénierie indiquée dans la première colonne. Les tests sont effectués durant 80 min d'incubation avec 0.01-0.02 mg de protéine d'extrait soluble de levure dans un volume réactionnel de 200 microL contenant 2 mM de ribulose diphosphate à température ambiante. Les activités sont données en nmoles de 3-phosphoglycérate formé/min/mg de protéines totales dans l'extrait.**

| | Souches | A | B | C |
|---|---|---|---|---|
| **CEN.PK n°3** | **RbcS/RbcL/RbcX/PRK/(GroES/GroEL) *E.coli*** | **20** | **13** | **20** |
| **CEN.PK n°2** | **RbcS/RbcL/RbcX/(GroES/GroEL) *E.coli*** | **ND** | **5** | **2,5** |
| **CEN.PK n°101** | **RbcS/RbcL/RbcX/PRK/(GroES/GroEL2) *S.elongatus*** | **ND** | **ND** | **1,5** |

### 3.3. La RuBisCO synthétique incorpore le CO₂ marqué au ¹³C pour l'intégrer dans le produit de réaction

L'expérience d'incorporation isotopique décrite plus haut (Figure 4) illustre par quantification de 3-glycerophosphate marqué la capacité du complexe RuBisCO à produire du 3-glycerophosphate marqué à partir de ribulose diphosphate, en fixant le carbone à partir de molécules de bicarbonate marquées au ¹³C

### 3.4. L'activité RuBisCO est augmentée par la présence de l'anhydrase carbonique

L'anhydrase carbonique, en catalysant l'inter conversion du bicarbonate en dioxyde de carbone solvaté, est un cofacteur connu de la réaction. Cet exemple confirme le comportement attendu pour une telle réaction. De manière intéressante, des tests d'activité *in vitro* montrent que l'ajout d'anhydrase carbonique bovine additionnée à une concentration finale de 10µg/ml dans le volume réactionnel d'un test d'activité RuBisCO, précédemment décrit, augmente le potentiel du complexe RuBisCO de trois à quatre fois (Figure 10). Cela suggère que l'optimisation de la concentration en CO₂ autour du complexe majore significativement les activités observées dans le test précédent, qui représente donc une valeur minimale pour les activités reconstituées. D'autres facteurs pourraient aussi y contribuer *in vivo*, les valeurs mesurées ne sont donc que des valeurs planchers.

### EXEMPLE 4 : CARACTERISATION PHENOTYPIQUE DES SOUCHES CONTENANT L'INGENIERIE

### 4.1. La culture anaérobie montre une augmentation de la production d'éthanol

Les précultures ont été réalisées sur milieu chimiquement défini. Après décongélation, 1 mL d'un tube du stock (- 80°C) a été prélevé pour ensemencer un flacon pénicilline (100 mL) contenant 10 mL de milieu de culture (dont 0.1g/L d'acide formique additionnée à 20g/L de glucose), incubé 18 heures à 30 °C et 120 rpm. Les précultures ont été réalisées en anaérobiose (flacons préalablement flushés à l'azote) et en présence de doxycycline (2µ/mL) afin d'éviter les problèmes de toxicité observés en présence du gène PRK.

Les précultures ont ensuite été lavées trois fois (centrifugation, resuspension, vortex 15 s) à l'eau physiologique (NaCl, 9 g L⁻¹), puis le culot cellulaire a été resuspendu dans du milieu de culture sans doxycycline.

Ces cellules issues des précultures ont ensuite été inoculées afin d'atteindre une densité optique initiale de 0,05 (soit 0,1 g L⁻¹). Le volume de départ des cultures était de 50 mL en aérobiose (fioles d'Erlenmeyer bafflées de 250 mL) ou de 35 mL en anaérobiose (flacons pénicilline de 100 mL).

Les cultures ont été arrêtées après consommation totale du glucose ou arrêt de la production d'éthanol.

La culture anaérobie a permis de caractériser phénotypiquement des souches contenant l'ingénierie complète CARBOYEAST ou des éléments isolés, de manière à caractériser l'influence de chacun sur la levure.

La figure 11 et le tableau XI montrent que, lors de cultures anaérobies sur glucose, l'ingénierie complète CARBOYEAST (RuBisCO_PRK_Chaperonnes) soit (RbcS+RbcL+PRK+RbcX+(GroES+GroEL) *E. coli*) induit une amélioration de la production d'éthanol, par rapport à une souche identique n'intégrant pas le cycle de Calvin (RbcX+(GroES+GroEL) *E. coli*). Le rendement en éthanol produit sur glucose consommé est ainsi amélioré de 7% (0,49 g/g *vs* 0,46 g/g), au détriment du rendement en biomasse (0,035 *vs.* 0,051 g /g), suggérant une redistribution accentuée du carbone vers la production d'éthanol.

**Tableau XI : Rendements de production d'éthanol et de biomasse lors de cultures anaérobies (Prk : phosphoribulokinase)**

| Génotype | | | Rendements | |
|---|---|---|---|---|
| | | | Biomasse | Ethanol |
| RuBisCO | PRK | Chaperonnes | g g⁻¹ | g g⁻¹ |
| - | - | + | 0,051 | 0,46 |
| + | + | + | 0,035 | 0,49 |

### 4.2. Etude de la fonctionnalité du complexe RuBisCO in vivo Protocole expérimental

Les précultures ont été réalisées sur milieu chimiquement défini. Après décongélation, 1 mL d'un tube du stock (- 80°C) a été prélevé pour ensemencer un flacon pénicilline (100 mL) contenant 10 mL de milieu de culture (dont 0.1g/L d'acide formique additionnée à 20g/L de glucose), incubé 18 heures à 30 °C et 120 rpm. Les précultures ont été réalisées en anaérobiose (flacons préalablement flushés à l'azote) et en présence de doxycycline (2p/mL) afin d'éviter les problèmes de toxicité observés en présence du gène PRK.

Les précultures ont ensuite été lavées trois fois (centrifugation, resuspension, vortex 15 s) à l'eau physiologique (NaCl, 9 g L⁻¹), puis le culot cellulaire a été resuspendu dans du milieu de culture sans doxycycline.

Ces cellules issues des précultures ont ensuite été inoculées dans le milieu de culture contenant 0.5g/L d'acide formique et 0.5g/L de glucose. Le volume de départ des cultures était de 25 mL (fioles d'Erlenmeyer bafflées de 250 mL).

Les différentes souches de levure sont cultivées sur du formate marqué ou non au ¹³C, additionné ou non de glucose non marqué. Pour démontrer l'incorporation d'isotope du carbone à partir du formate, la composition isotopique d'un métabolite cellulaire stable, l'ergosterol, est analysée. Les cultures cellulaires ont été centrifugées 5 min à 10000 *rpm* et le culot resuspendu dans 7mL Chloroforme/Méthanol (2/1) et centrifugé 5 min à 10000 *rpm.* Le surnageant est additionné de 2mL de TE, et après centrifugation de 5 min à 10000 *rpm* la phase chloroforme est récupérée et évaporée sous flux d'azote. Le reliquat est resuspendu dans 500pL de méthanol. Les échantillons sont analysés par chromatographie liquide à haute performance (C.L.H.P.) sur un chromatographe (Waters, Alliance 2690) équipé d'une colonne Aminex HPX 87-H⁺ (300 mm × 7,8 mm).

### Résultats

Le transport du CO₂ dans la levure de l'extérieur vers l'intérieur de la cellule n'étant pas un processus naturel et dans l'attente d'une ingénierie complémentaire permettant de l'établir par co-expression d'un transporteur tel que les aquaporines spécialisées décrites chez *S. elongatus,* de l'acide formique pouvant être oxydé par une déshydrogénase de la levure en dioxyde de carbone a été utilisé comme source de dioxyde de carbone intracellulaire. Ce dioxyde de carbone peut potentiellement être réincorporé en matières organiques au travers du complexe RuBisCO. Ainsi, en présence de formate marqué au ¹³C, une incorporation de l'isotope dans la biomasse est attendue. Néanmoins, l'existence d'autres réactions naturelles anaplérotiques (capables de fixer du CO₂) chez la levure explique que dans ces conditions on a observé un bruit de fond important d'incorporation du ¹³C (de l'ordre de 3-4% de marquage) même en absence du complexe RuBisCO, rendant ambiguë l'interprétation de la contribution de la RuBisCO dans l'incorporation isotopique observée. Une analyse des schémas métaboliques montre que les conditions utilisées dans cette première expérience ne sont en fait pas adaptées à une mesure isotopique de l'activité RuBisCO *in vivo.* A noter que cette expérience a permis néanmoins de confirmer que l'absence d'incorporation *in vivo* du bicarbonate marqué lorsqu'il est ajouté dans le milieu de culture en utilisant le glucose et non le formate comme source de carbone est bien dû à un problème de transport du CO₂ (ou du bicarbonate/carbonate) et non à un problème métabolique.

En conséquence, notre attention s'est portée sur d'autres éléments de preuve de concept comme la cinétique de consommation de l'acide formique et le maintien de la viabilité des souches portant ou non l'ingénierie. A noter que l'utilisation d'acide formique comme seule source de carbone ne permet pas la croissance de la souche du fait de ressources énergétiques insuffisantes, au moins en l'absence d'ingénierie complémentaire des formates déshydrogénases. Seul le maintien de viabilité est observable dans ces conditions. Ce bilan énergétique peut néanmoins être amélioré par ajout de faible quantité de glucose.

### Utilisation du formate comme source de carbone

Des cultures aérobies sur acide formique (0,45 g/l) et glucose (0,55 g/l) ont permis de caractériser phénotypiquement des souches contenant l'ingénierie complète CARBOYEAST ou des éléments isolés de manière à caractériser la croissance sur acide formique. L'acide formique peut être métabolisé chez la levure en CO₂ et pouvoir réducteur (H₂) par la formate déshydrogénase, néanmoins la levure n'est pas capable de pousser sur acide formique comme seule source de carbone.

La figure 12 montre que, lors de cultures aérobies, l'ingénierie complète CARBOYEAST (CEN.PK n°3 : RbcS+RbcL+PRK+RbcX+(GroES+GroEL)*E.coli*) induit une consommation complète de l'acide formique, par rapport à une souche identique, dépourvue de PRK (CEN.PK n°2 : RbcS+RbcL+RbcX+(GroES+GroEL)E.coli), ne permettant pas la production de substrat pour la RuBisCO. En parallèle, une amélioration de la production de biomasse est observée, alors que la consommation de glucose demeure identique. La souche présentant l'ingénierie complète présente donc une meilleure capacité de transformation de l'acide formique.

### 4.3. L'introduction chez la levure d'un cycle de Calvin dépendant de la RuBisCO modifie in vivo l'équilibre des voies de biosynthèse dans le métabolisme central

L'objet de cette étude est de démontrer que l'introduction d'un cycle de Calvin chez la levure par coexpression fonctionnelle de la RuBisCO (et des chaperonnes) et de la phosphoribulokinase modifie significativement le profil métabolique interne dans un sens compatible avec la fonctionnalité de l'ingénierie *in vivo.* Ce profil métabolique a été évalué après culture de souches portant une ingénierie complète ou seulement partielle et analyse comparative du phospho-métabolome par spectrométrie de masse couplée à la chromatographie HPLC (chromatographie phase reverse en appariement d'ions).

Les souches testées sont : La souche contenant l'ingénierie complète (CEN.PK n°3) et celle dépourvue de PRK (CEN.PK n°2). Les cellules sont cultivées à 30°C à l'air ambiant avec agitation sur milieu YNB (*yeast without nitrogen base*), supplémenté en sulfate d'ammonium 6,7 g.L⁻¹, glucose 20 g.L⁻¹, agar 20 g.L⁻¹ pour les géloses) avec un milieu commercial CSM (MP Biomedicals), et adapté aux marqueurs de sélection des plasmides utilisés (milieu sans leucine, sans uracile et sans tryptophane). Les cultures sont arrêtées par refroidissement à 4°C une génération avant la fin de la phase exponentielle. L'analyse se fait sur des extractions protéiques issues de 1mL de cellules en phase exponentielle de croissance quenchée avec 5 mL de methanol/eau 80% (v/v) +10mM AcNH₄. Après centrifugation, le culot est conservé à -80°C. L'extraction se fait en suspendant le culot dans 5mL d'ethanol/eau 75% (v/v), 10mM AcNH₄ avec ajout extemporané de 150pL d'un mélange de standards de métabolites purs marqués au ¹³C (méthode IDMS). Après une incubation de 5 min à 80°C et refroidissement rapide dans un bain d'azote liquide, une centrifugation permet d'éliminer les débris.

La méthode IDMS est utilisée pour permettre une quantification absolue. Dans le cadre de cette analyse, la quantification absolue du ribulose-1,5-bisphosphate n'a pu être obtenue faute de disponibilité de standard adéquat et a été remplacée par une calibration externe non isotopique qui permet néanmoins une estimation (probablement sous-évaluée) de la concentration de ce composé chez la levure.

Les résultats, présentés dans la figure 13, suggèrent un rééquilibre des voies de synthèses au sein de la cellule prenant en compte le Cycle de Calvin reconstitué, que l'on pourrait résumer par la simulation métabolique de la figure 14.

## Revendications

1. Cellule de levure transformée, **caractérisée en ce qu'**elle contient :
a) une cassette d'expression contenant une séquence codant pour la sous-unité RbcL d'une enzyme RuBisCO de forme I de cyanobactérie du genre Synechococcus, sous contrôle transcriptionnel d'un promoteur approprié ;
b) une cassette d'expression contenant une séquence codant pour la sous-unité RbcS de ladite enzyme RuBisCO, sous contrôle transcriptionnel d'un promoteur approprié ;
c) une cassette d'expression contenant une séquence codant pour la chaperonne spécifique RbcX de ladite enzyme RuBisCO, sous contrôle transcriptionnel d'un promoteur approprié, dans laquelle RbcX est une chaperonne de cyanobactérie présentant plus de 50% d'identité avec SEQ ID N°3 et conservant une activité de chaperonne RbcX de S. elongatus ;
d) une cassette d'expression contenant une séquence codant pour une chaperonne GroES de E. coli, sous contrôle transcriptionnel d'un promoteur approprié ; et
e) une cassette d'expression contenant une séquence codant pour une chaperonne GroEL de E. coli, sous contrôle transcriptionnel d'un promoteur approprié.

2. Cellule selon la revendication 1, **caractérisée en ce que** la cassette d'expression contenant la séquence codant pour la sous-unité RbcL comprend la séquence SEQ ID NO : 1, la cassette d'expression contenant la séquence codant pour la sous-unité RbcS comprend la séquence SEQ ID NO :2 et la cassette d'expression contenant la séquence codant pour la chaperonne RbcX comprend la séquence SEQ ID NO :3.

3. Cellule selon l'une quelconque des revendications 1 à 2, **caractérisée en ce que** les trois cassettes d'expression mentionnées aux points c), d) et e) de la revendication 1 forment un bloc continu d'information génétique.

4. Cellule selon l'une quelconque des revendications 1 à 3, **caractérisée en ce que** les cassettes d'expression mentionnées aux points c), d) et e) de la revendication 1 sont portées par un élément génétique épisomique unique.

5. Cellule de levure selon l'une quelconque des revendications 1 à 4, **caractérisée en ce que** ladite levure appartient à l'espèce *Saccharomyces cerevisiae.*

6. Cellule de levure selon l'une quelconque des revendications 1 à 5, **caractérisée en ce qu'**elle contient en outre une cassette d'expression contenant une séquence codant pour une phosphoribulokinase (PRK) sous contrôle transcriptionnel d'un promoteur approprié.

7. Cellule de levure selon la revendication 6, **caractérisée en ce que** ladite PRK est une PRK de classe II.

8. Cellule de levure selon la revendication 7, **caractérisée en ce que** ladite PRK de classe II est choisie parmi les PRK de *Spinnacia oleacera, Euglena gracilis,* ou *Synechococcus elongatus.*

9. Cellule de levure selon l'une quelconque des revendications 6 à 8, **caractérisée en ce que** le promoteur contrôlant la transcription de la séquence codant pour la PRK est un promoteur inductible.

10. Procédé de production de biomasse à partir de dioxyde de carbone comprenant une étape de mise en culture d'une cellule de levure selon l'une quelconque des revendications 1 à 9 en présence d'une source de carbone et d'une source de dioxyde de carbone.

11. Procédé de production de composés organiques à partir de dioxyde de carbone comprenant une étape de mise en culture d'une cellule de levure selon l'une quelconque des revendications 1 à 9 en présence d'une source de carbone et d'une source de dioxyde de carbone.

## Patentansprüche

1. Transformierte Hefezelle, **dadurch gekennzeichnet, dass** sie Folgendes enthält:
a) eine Expressionskassette, die eine Sequenz enthält, die die RbcL-Untereinheit eines RuBisCO-Enzyms der Form I von Cyanobakterien der Gattung Synechococcus, unter transkriptionaler Kontrolle eines geeigneten Promotors enthält;
b) eine Expressionskassette, die eine Sequenz enthält, die die RbcS-Untereinheit des RuBisCO-Enzyms unter transkriptionaler Kontrolle eines geeigneten Promotors codiert;
c) eine Expressionskassette, die eine Sequenz enthält, die unter transkriptionaler Kontrolle eines geeigneten Promotors für das RbcX-spezifische Chaperon des RuBisCO-Enzyms codiert, wobei RbcX ein Cyanobakterium-Chaperon ist, das über 50% Identität mit SEQ ID NR: 3 aufweist und eine Aktivität des RbcX-Chaperons von S. elongatus aufrechterhält;
d) eine Expressionskassette, die eine Sequenz enthält, die für ein E. coli GroES-Chaperon unter transkriptionaler Kontrolle eines geeigneten Promotors codiert; und
e) eine Expressionskassette, die eine Sequenz enthält, die für ein E. coli GroEL-Chaperon unter transkriptionaler Kontrolle eines geeigneten Promotors codiert.

2. Zelle nach Anspruch 1, **dadurch gekennzeichnet, dass** die Expressionskassette, die die Sequenz enthält, die für die RbcL-Untereinheit codiert, die Sequenz SEQ ID NR: 1 umfasst, wobei die Expressionskassette, die die Sequenz enthält, die für die RbcS-Untereinheit codiert, die Sequenz SEQ ID NO: 2 umfasst und die Expressionskassette, die die Sequenz enthält, die für das RbcX-Chaperon codiert, die Sequenz SEQ ID NO: 3 umfasst.

3. Zelle nach einem der Ansprüche 1 bis 2, **dadurch gekennzeichnet, dass** die drei unter den Punkten c), d) und e) des Anspruchs 1 genannten Expressionskassetten einen kontinuierlichen Block von genetischer Information bilden.

4. Zelle nach einem der Ansprüche 1 bis 3, **dadurch gekennzeichnet, dass** die unter den Punkten c), d) und e) des Anspruchs 1 genannten Expressionskassetten von einem einzigen episomischen genetischen Element getragen werden.

5. Hefezelle nach einem der Ansprüche 1 bis 4, **dadurch gekennzeichnet, dass** die Hefe zu der Art *Saccharomyces cerevisiae* gehört.

6. Hefezelle nach einem der Ansprüche 1 bis 5, **dadurch gekennzeichnet, dass** sie ferner eine Expressionskassette enthält, die eine Sequenz enthält, die eine Phosphoribulokinase (PRK) unter transkriptionaler Kontrolle eines geeigneten Promotors codiert.

7. Hefezelle nach Anspruch 6, **dadurch gekennzeichnet, dass** die PRK eine PRK der Klasse II ist.

8. Hefezelle nach Anspruch 7, **dadurch gekennzeichnet, dass** die PRK der Klasse II unter den PRK von *Spinnacia oleacera, Euglena gracilis,* oder *Synechococcus elongatus* ausgewählt ist.

9. Hefezelle nach einem der Ansprüche 6 bis 8, **dadurch gekennzeichnet, dass** der Promotor, der die Transkription der Sequenz steuert, die für die PRK codiert, ein induzierbarer Promotor ist.

10. Verfahren zur Herstellung von Biomasse ausgehend von Kohlendioxid, umfassend einen Schritt der Inkulturnahme einer Hefezelle nach einem der Ansprüche 1 bis 9 in Gegenwart einer Kohlenstoffquelle und einer Kohlendioxidquelle.

11. Verfahren zur Herstellung von organischen Verbindungen aus Kohlendioxid, umfassend einen Schritt der Inkulturnahme einer Hefezelle nach einem der Ansprüche 1 bis 9 in Gegenwart einer Kohlenstoffquelle und einer Kohlendioxidquelle.

## Claims

1. Transformed yeast cell, **characterized in that** it contains:
(a) an expression cassette containing a sequence encoding the RbcL subunit of a form I RuBisCO enzyme from a cyanobacterium of the genus Synechococcus, under the transcriptional control of a suitable promoter;
b) an expression cassette containing a sequence encoding the RbcS subunit of said RuBisCO enzyme, under the transcriptional control of a suitable promoter;
c) an expression cassette containing a sequence encoding the specific chaperone RbcX of said RuBisCO enzyme, under the transcriptional control of a suitable promoter, wherein RbcX is a cyanobacterial chaperone having more than 50% identity with SEQ ID NO: 3 and retaining an S. elongatus chaperone RbcX activity;
(d) an expression cassette containing a sequence encoding an E. coli chaperone GroES, under the transcriptional control of a suitable promoter; and
(e) an expression cassette containing a sequence encoding an E. coli chaperone GroEL, under the transcriptional control of a suitable promoter.

2. Cell according to claim 1, **characterized in that** the expression cassette containing the sequence encoding the RbcL subunit comprises SEQ ID NO: 1, the expression cassette containing the sequence encoding the RbcS subunit comprises SEQ ID NO: 2 and the expression cassette containing the sequence encoding the chaperone RbcX comprises SEQ ID NO: 3.

3. Cell according to any one of claims 1 to 2, **characterized in that** the three expression cassettes mentioned in points c), d) and e) of claim 1 form a continuous block of genetic information.

4. Cell according to any one of claims 1 to 3, **characterized in that** the expression cassettes mentioned in points c), d) and e) of claim 1 are carried by a single episomal genetic element.

5. Yeast cell according to any one of claims 1 to 4, **characterized in that** said yeast belongs to the species *Saccharomyces cerevisiae.*

6. Yeast cell according to any one of claims 1 to 5, **characterized in that** it further contains an expression cassette containing a sequence encoding a phosphoribulokinase (PRK) under the transcriptional control of a suitable promoter.

7. Yeast cell according to claim 6, **characterized in that** said PRK is a class II PRK.

8. Yeast cell according to claim 7, **characterized in that** said class II PRK is selected from PRKs from *Spinnacia oleacera, Euglena gracilis,* or *Synechococcus elongatus.*

9. Yeast cell according to any one of claims 6 to 8, **characterized in that** the promoter controlling transcription of the sequence encoding the PRK is an inducible promoter.

10. Process for producing biomass from carbon dioxide comprising a step of culturing a yeast cell according to any one of claims 1 to 9 in the presence of a carbon source and a carbon dioxide source.

11. Process for producing organic compounds from carbon dioxide comprising a step of culturing a yeast cell according to any one of claims 1 to 9 in the presence of a carbon source and a carbon dioxide source.
